(19) Europäisches Patentamt

European Patent Office

Office européen des brevets

(11) **EP 0 585 492 B1**

(12) # EUROPEAN PATENT SPECIFICATION

(45) Date of publication and mention
of the grant of the patent:
**27.01.1999 Bulletin 1999/04**

(51) Int Cl.$^6$: **G01N 29/18, A61B 8/08**

(21) Application number: **92202509.3**

(22) Date of filing: **15.08.1992**

(54) **Apparatus for determining the mechanical properties of a solid**

Vorrichtung zur Bestimmung der mechanischen Eigenschaften eines Festkörpers

Appareil pour déterminer les propriétés mécaniques d'un solide

(84) Designated Contracting States:
**AT BE CH DE DK ES FR GB GR IE IT LI LU MC NL PT SE**

(43) Date of publication of application:
**09.03.1994 Bulletin 1994/10**

(73) Proprietor: **MEDICANO SYSTEMS LTD.**
**Rehovot (IL)**

(72) Inventors:
- **Kantorovich, Edward**
**Tel Aviv (IL)**
- **Rapoport, Alex**
**Rishon Lezion (IL)**

(74) Representative: **Ungria Lopez, Javier et al**
**Avda. Ramon y Cajal, 78**
**28043 Madrid (ES)**

(56) References cited:
| | |
|---|---|
| **WO-A-90/01903** | **US-A- 3 720 098** |
| **US-A- 4 372 163** | **US-A- 4 398 421** |
| **US-A- 4 413 517** | **US-A- 5 143 072** |

- **JAPANESE JOURNAL OF APPLIED PHYSICS, SUPPLEMENTS vol. 27-1, March 1988, TOKYO JA pages 160 - 162 T. NOMURA ET AL: 'Precise measurement of surface acoustic wave velocity using a swept frequency IDT-NDE system'**

## Description

### FIELD OF THE INVENTION

The present invention relates to instrumentation for non-destructive measurement of mechanical properties of materials generally and to instrumentation for non-invasive measurement of the mechanical properties of bone and bone quality.

### BACKGROUND OF THE INVENTION

It is known in the art that the velocity of a sound wave in a material depends on the mechanical properties of the material. This is outlined by C.H. Hastings and S. W. Carter in an article entitled "Inspection, Processing and Manufacturing Control of Metal by Ultrasonic Methods", Symposium on Ultrasonic Testing, 52nd Annual Meeting of the American Society for Testing Materials, June 28, 1949, pp. 16 - 47.

U.S. Patents 3,720,098, 3,228,232, 3,288,241, 3,372,163, 3,127,950, 3,512,400, 4,640,132, 4,597,292 and 4,752,917 describe the state of the art of non-destructive testing.

A sound wave which reaches a semi-infinite solid at an angle will typically propagate through the solid as three waves, namely, the longitudinal, transverse and surface waves, wherein each wave has a different velocity. As described by Hastings and Carter, the velocities of the three waves are defined as follows:

$$V_L = \sqrt{\frac{E\,(1 - \sigma)}{rho\,(1 + \sigma)(1 - 2\sigma)}} \tag{1}$$

$$V_T = \sqrt{\frac{E}{2\,(1 + \sigma)\,rho}} \tag{2}$$

$$V_S = \alpha\,V_T \tag{3a}$$

$$\alpha = \frac{0.87 + 1.12\,\sigma}{1 + \sigma} \tag{3b}$$

where $V_L$, $V_T$, and $V_S$ are, respectively, the velocities of the longitudinal, transverse and Rayleigh surface waves, and E, $\sigma$ and rho are, respectively, the Young's Modulus, the Poisson's ratio of lateral contraction to longitudinal extension and the mass density of the material. Equation 3b is an empirical relationship as defined on page 326 of Wave Motion in Elastic Solids, by Karl F. Graff, published by the Clarendon Press, Oxford England in 1975.

In ultrasonic measurement of the condition of bone, typically only the velocity of the longitudinal wave is used. As defined in the article, "Osteoporotic Bone Fragility: Detection by Ultrasound Transmission Velocity," R.P. Heaney et al., JAMA, Vol. 261, No. 20, May 26, 1989, pp. 2986 - 2990, the Young's modulus of a bone E is given empirically as:

$$E = K\,(rho)^2 \tag{4a}$$

and the velocity of sound through the bone is a function of E, where the velocity of sound is typically the longitudinal velocity, as follows:

$$V_L = \sqrt{(E/rho)} = \sqrt{(K * rho)} \tag{4b}$$

where K is a constant which incorporates a number of factors, such as spatial orientation of the bone structures, inherent properties of the bone material and fatigue damage. Thus, the velocity of a longitudinal wave is a function of the mass density and can be used as an indicator of the quality of bone.

The following articles also discuss ultrasonic measurement of bone condition both in vivo and in vitro.

"Measurement of the Velocity of Ultrasound in Human Cortical Bone In Vivo," M.A. Greenfield, et al., Radiology, Vol. 138, March 1981, pp. 701 - 710.

"Combined 2.25 MHz ultrasound velocity and bone mineral density measurements in the equine metacarpus and their in vivo applications," R.N. McCartney and L.B. Jeffcott, Medical and Biological Engineering and Computation, Vol. 25, 1987, Nov. 1877, pp. 620 - 626.

In order to perform in vivo ultrasonically measurements of the mechanical properties of bone, it is necessary to transmit an ultrasonic wave through the soft tissue surrounding the bone. Unfortunately, the thickness of the soft tissue varies along the length of the bone. This can affect the accuracy of the ultrasound propagation time measurement through the bone. In the abovementioned articles, the thickness of the soft tissue is either ignored or an attempt is made to cancel the effects of the soft tissue. In the articles describing in vitro experiments, the soft tissue is removed from the bone.

Russian patents 1,420,383, 1,308,319, 1,175,435, 1,324,479, 1,159,556 and 1,172,534 and U.S. Patents 4,926,870, 4,361,154, 4,774,959, 4,421,119, 4,941,474, 3,847,141, 4,913,157 and 4,930,511 describe various systems for measuring the strength of bone based on the velocity $V_L$. These systems typically have one ultrasonic signal transmitter and at least one ultrasonic signal receiver.

Russian patents 1,420,383, 1,308,319 and 1,175,435 solve the problem of the unknown thickness of the soft tissue by assuming values for the thickness of the soft tissue in the area of the measurement or by assuming that the thickness variation is small over the distance between two ultrasonic signal receivers.

Russian patent 1,342,279 utilizes two receivers and a single transmitter and calculates an average group speed through the bone based on the known distance between the two receivers.

Russian patent 1,159,556 defines zones of a bone and the condition of a bone is determined by the difference between the maximum and minimum amplitude of the ultrasound signals measured. It would appear that this measurement is performed on an excised bone.

Russian patent 1,172,534 describes a system which compares the ultrasound signal of a healthy bone with that of an unhealthy bone and from the comparison, produces a diagnosis of the extent of disease in the unhealthy bone.

U.S. Patents 4,926,870, 4,421,119 and 3,847,141 describe systems which places a receiver and a transmitter on opposite sides of a bone. U.S. Patent 4,926,870 also compares the resultant signal with a canonical waveform, thereby to identify the health of the bone.

U.S. Patents 4,913,157, 4,774,959 and 4,941,474 describe systems which transmit an ultrasonic signal with a spectrum of frequencies.

U.S. Patent 4,930,511 describes a system is placed around a standard inanimate homogeneous material of known acoustic properties before it is placed around a bone.

## SUMMARY OF THE INVENTION

The present invention ultrasonically measures mechanical properties of a hard material wherein the ultrasonic signal travels from a transmitter, through a thickness of an interposed medium and through a hard material to be tested, such as a solid. The ultrasonic wave propagates through the hard material as three waves, the longitudinal, transverse and surface waves. From the solid, the ultrasonic wave travels through a second thickness to a first receiver and through a third thickness to a second receiver located a defined distance from the first receiver.

There is therefore provided, in accordance with an embodiment of the present invention, apparatus for determining, through an interposed medium, the mechanical properties of a solid having a surface. The apparatus includes a) ultrasonic transmission apparatus, located in a first location, for transmitting ultrasonic waves through the interposed medium and the solid, generally parallel to the surface, b) at least one ultrasonic receiver unit, located in each of second and third locations, for receiving the ultrasonic waves, wherein the first, second and third locations are colinear along the surface, c) apparatus for locating the receiver unit such that a first receipt time of an ultrasonic wave from the surface to the receiver unit at the second location is generally equivalent to a second receipt time from the surface to the receiver unit at the third location, and d) apparatus for calculating the mechanical properties from ultrasonic waves transmitted by the ultrasonic transmission apparatus once the first and second receipt times are generally identical.

Additionally, in accordance with an embodiment of the present invention, the apparatus for locating adjusts the locations of the at least one receiver unit such that the first and second receipt times are generally equivalent. The apparatus for locating also includes apparatus for determining the first and the second receipt times.

Furthermore, in accordance with an embodiment of the present invention, the apparatus for determining includes apparatus for pulsing the transmission apparatus thereby to transmit ultrasonic waves when the first and second receipt times are generally identical.

Still further, in accordance with an embodiment of the present invention, the solid is in vivo bone. Alternatively, the solid is selected from the group of metal, plastics and wood.

Moreover, in accordance with an embodiment of the present invention, the velocity of the ultrasonic waves through the interposed medium is less than the velocity of the ultrasonic waves through the solid.

Additionally, in accordance with an embodiment of the present invention, the at least one ultrasonic receiver unit is placed onto a surface of the interposed medium generally without any coupling medium.

There is further provided, in accordance with an embodiment of the present invention, apparatus for determining, through an interposed medium, the mechanical properties of a solid. The apparatus includes a) ultrasonic transmission apparatus for transmitting an ultrasonic wave through the interposed medium and the solid, wherein the solid has a surface, b) at least first and second ultrasonic receiver units for receiving the ultrasonic wave and c) apparatus for locating the ultrasonic receiver units including a rocker unit for rockingly pressing the ultrasonic receiver units into the interposed medium such that a first receipt time of the ultrasonic wave from the surface to the first ultrasonic receiver unit is generally equivalent to a second receipt time from the surface to the second ultrasonic receiver unit. The apparatus for locating adjusts the locations of the receiver units such that the first and second receipt times are generally equivalent. The rocker unit typically includes an acoustically insulating unit.

Still further, in accordance with an embodiment of the present invention, the ultrasonic transmission apparatus includes apparatus for transmitting the ultrasonic wave within a wide angle and the ultrasonic wave generates longitudinal, surface and transverse waves in the solid.

Moreover, in accordance with an embodiment of the present invention, the apparatus includes processing apparatus for processing signals from the receiver unit and for identifying a receipt time of the longitudinal wave. The processing apparatus includes apparatus for identifying receipt times of the surface and transverse waves.

Additionally, in accordance with an embodiment of the present invention, the processing apparatus includes apparatus for determining the mechanical properties from at least the receipt time for the longitudinal wave. Alternatively or in addition, the processing apparatus includes apparatus for determining the mechanical properties from the receipt times of the surface and transverse waves.

Further, in accordance with an embodiment of the present invention, the transmission apparatus transmits the ultrasonic wave within a generally small range of a predetermined angle.

Still further, in accordance with an embodiment of the present invention, a transmission surface of the transmission apparatus and effective receiving locations of the receiving apparatus are generally coplanar.

Moreover, in accordance with an embodiment of the present invention, the apparatus is characterized in that generally no knowledge of properties of the interposed medium are necessary.

There is also provided, in accordance with an embodiment of the present invention, a method for determining, through an interposed medium, the mechanical properties of a solid having a surface. The method includes the steps of transmitting ultrasonic waves through the interposed medium and through the solid and generally parallel to the surface, from a first location, receiving the ultrasonic wave with at least one ultrasonic receiver unit located in each of second and third locations, wherein the first, second and third locations are colinear along the surface, locating the receiver unit such that a first receipt time of an ultrasonic wave from the surface to the receiver unit at the second location is generally equivalent to a second receipt time from the surface to the receiver unit at the third location, and calculating the mechanical properties from ultrasonic waves transmitted by the ultrasonic transmission apparatus once the first and second receipt times are generally equivalent.

Additionally, in accordance with an embodiment of the present invention, the step of locating includes the step of adjusting the locations of the receiver unit such that the first and second receipt times are generally equivalent.

Further, in accordance with an embodiment of the present invention, the step of locating includes the step of determining the first and the second receipt times. The step of determining also includes the step of pulsing the transmission apparatus thereby to transmit ultrasonic waves when the first and second receipt times are generally identical.

Moreover, in accordance with an embodiment of the present invention, the solid is in vivo bone. Alternatively, the solid is selected from the group of metal, plastics and wood.

Additionally, in accordance with an embodiment of the present invention, the velocity of the ultrasonic waves through the interposed medium is less than the velocity of the ultrasonic waves through the solid.

Furthermore, in accordance with an embodiment of the present invention, the ultrasonic receiver unit is placed onto a surface of the interposed medium generally without any coupling medium.

There is also provided, in accordance with an embodiment of the present invention, a method for determining, through an interposed medium, the mechanical properties of a solid. The method comprises the steps of a) transmitting an ultrasonic wave through the interposed medium and the solid, wherein the solid has a surface, b) receiving the ultrasonic wave with at least first and second ultrasonic receiver units and c) locating the receiver units such that a first receipt time of the ultrasonic wave from the surface to the first receiver unit is generally equivalent to a second receipt time from the surface to the second receiver unit. The step of locating includes the step of using a rocker unit to rockingly press the ultrasonic receiver units into the interposed medium thereby adjusting the locations of the receiver units such that the first and second receipt times are generally equivalent.

Additionally, in accordance with an embodiment of the present invention, the method includes the step of transmitting the ultrasonic waves in a wide band pattern and the ultrasonic waves generate longitudinal, surface and transverse waves in the solid.

Moreover, in in accordance with an embodiment of the present invention, the method includes the steps of processing signals from the receiver apparatus and identifying a receipt time of the longitudinal wave.

Additionally, in accordance with an embodiment of the present invention, the step of processing includes the step of identifying receipt times of the surface and transverse waves. The step of processing preferably includes the step of determining the mechanical properties from at least the receipt time for the longitudinal wave. Alternatively, or in addition, the step of processing includes the step of determining the mechanical properties from the receipt times of the surface and transverse waves.

Further, in accordance with an embodiment of the present invention, the solid comprises more than one material and the apparatus for calculating includes apparatus for receiving a time-based output signal from each of the at least one ultrasonic receiver unit, apparatus for transforming at least two of the output signals to the frequency domain thereby to produce frequency domain signals and apparatus for determining, from the frequency domain signals, frequency values and their corresponding propagation speeds, for each of the materials within the solid.

Still further, in accordance with an embodiment of the present invention, the apparatus additionally includes apparatus for determining, from a difference of the frequency domain signals, extent of attenuation of each of the materials within the solid. Alternatively or in addition, the apparatus also includes apparatus for receiving a time-based output signal from each of the at least one ultrasonic receiver unit and determining a first peak of each of the output signals and apparatus for calculating from the first peaks, the extent of attenuation of the ultrasonic waves by the solid.

Moreover, in accordance with an embodiment of the present invention, the apparatus for calculating includes apparatus for estimating a thickness of the solid.

Additionally, in accordance with an embodiment of the present invention, when the solid comprises more than one material the step of calculating includes the steps of receiving a time-based output signal from each of the at least one ultrasonic receiver unit, transforming at least two of the output signals to the frequency domain thereby to produce frequency domain signals and determining, from the frequency domain signals, frequency values and their corresponding propagation speeds, for each of the materials within the solid.

Further, in accordance with an embodiment of the present invention, the method additionally includes the step of determining, from a difference of the frequency domain signals, extent of attenuation of each of the materials within the solid. Alternatively or in addition, the method also includes the steps of receiving a time-based output signal from each of the at least one ultrasonic receiver unit and determining a first peak of each of the output signals and calculating from the first peaks, the extent of attenuation of the ultrasonic waves by the solid.

Moreover, in accordance with an embodiment of the present invention, the step of calculating includes the step of estimating a thickness of the solid.

Furthermore, in accordance with an embodiment of the present invention, the apparatus of the present invention can alternatively includes a housing and an acoustic barrier wherein the ultrasonic transmission apparatus are located on a first side of the acoustic barrier and the at least one ultrasonic receiver unit is located on a second side of the acoustic barrier.

Additionally, in accordance with an embodiment of the present invention, the apparatus for locating includes a housing in which the at least one ultrasonic receiver unit is located and a flexible membrane attached to the housing at one end and forming a closed section and an open section. The closed section is filled with a coupling material and the flexible membrane has a vacuum hole at a second end thereof. When the apparatus is placed onto human skin and air within the vacuum hole is evacuated, the apparatus is sealingly coupled with the human skin.

Further, there is provided, in accordance with an embodiment of the present invention, apparatus for determining, through an interposed medium, the mechanical properties of a solid having a surface. The apparatus includes a flexible array of piezoelectric cells formable into at least one ultrasonic transmitter and at least one ultrasonic receiver, and apparatus for defining a first plurality of the piezoelectric cells in a first location as the at least one ultrasonic transmitter and at least second and third pluralities of piezoelectric cells, in second and third locations, respectively, as the at least one ultrasonic receiver. The first, second and third locations are colinear along the surface, and the ultrasonic transmitter transmits ultrasonic waves through the interposed medium and through the solid in a direction generally parallel to the surface. The apparatus also includes apparatus for calculating the mechanical properties from ultrasonic waves transmitted by the ultrasonic transmitter. The apparatus preferably also includes apparatus for defining the at least one receiver unit such that a first receipt time of an ultrasonic wave from the surface to the at least one ultrasonic receiver unit at the second location is generally equivalent to a second receipt time from the surface to the at least one ultrasonic receiver unit at the third location.

Finally, there is provided, in accordance with an embodiment of the present invention, a method of scanning, through an interposed medium, a solid having a surface and for determining therefrom the mechanical properties of the solid. The method comprises the steps of a) defining a first plurality of transducer cells in a first location as at least one ultrasonic transmitter and at least second and third pluralities of transducer cells, in second and third locations, respectively, as at least one ultrasonic receiver, wherein the first, second and third locations are colinear along the surface, and wherein the ultrasonic transmitter transmits ultrasonic waves through the interposed medium and through the solid in a direction generally parallel to the surface, b) calculating the mechanical properties from ultrasonic waves transmitted by the ultrasonic transmitter and c) repeating the steps of defining and calculating for different locations on the solid.

The method also preferably includes the step of defining the at least one receiver unit such that a first receipt time of an ultrasonic wave from the surface to the at least one ultrasonic receiver unit at the second location is generally equivalent to a second receipt time from the surface to the at least one ultrasonic receiver unit at the third location, and wherein the step of repeating is performed in randomly chosen locations.

Alternatively, in accordance with an embodiment of the present invention, the step of repeating is performed in sequentially chosen locations and wherein the step of calculating includes the step of determining the mechanical properties from knowledge of the velocity of the ultrasonic waves through the interposed medium.

## BRIEF DESCRIPTION OF THE DRAWINGS

The present invention will be understood and appreciated more fully from the following detailed description taken in conjunction with the drawings in which:

Fig. 1 is a schematic illustration of a method of ultrasonically measuring mechanical properties of materials operative in accordance with the present invention;

Figs. 2A and 2B are graphic illustrations of ultrasonic signals transmitted and received by ultrasonic transducers using the method of Fig. 1;

Fig. 3 is a part block diagram, part schematic illustration of in vivo ultrasonic apparatus for mechanical property measurement utilizing the method of Fig. 1;

Fig. 4 is a pictorial illustration of apparatus for ensuring that ultrasonic transducers are parallel to a surface of a material to be measured useful in the apparatus of Fig. 3;

Fig. 5 is a part block diagram, part schematic illustration of an alternative embodiment of the apparatus of Fig. 3;

Fig. 6 is a schematic illustration of an acoustically insulating element useful in the apparatus of Fig. 5;

Fig. 7 is a block diagram illustration of signal processors useful in the embodiments of Figs. 3 and 5;

Figs. 8A - 8D are graphical illustrations indicating the output signals from elements of the signal processor of Fig. 7;

Figs. 9A - 9C are schematic illustrations of transmitters useful in the embodiments of Figs. 3 and 5;

Fig. 10 is a flow chart illustration of a method of determining the attenuation of ultrasonic signals due to travel through the material;

Figs. 11A and 11B are graphical illustrations indicating output signals from the two ultrasonic receivers forming part of the apparatus of Fig. 3;

Fig. 12 is a graphical illustration of Fourier Transforms of the output signals of Figs. 11A and 11B;

Fig. 13 is a schematic illustration of a part formed of two materials;

Fig. 14 is a graphical illustration of the difference between the Fourier Transforms of Fig. 12;

Fig. 15 is a graphical illustration of the relationship between thickness of a part and the velocity of an ultrasonic wave through the part;

Fig. 16 is a block diagram illustration of a unit for determining attenuation from the signals shown in Figs. 11A and 11B;

Fig. 17 is a schematic side view illustration of a transmitter and a plurality of receivers combined into a single unit;

Figs. 18A and 18B are schematic side and front view illustrations respectively, of an alternative embodiment of a portion of the apparatus of Fig. 3.

## DETAILED DESCRIPTION OF PRESENT INVENTION

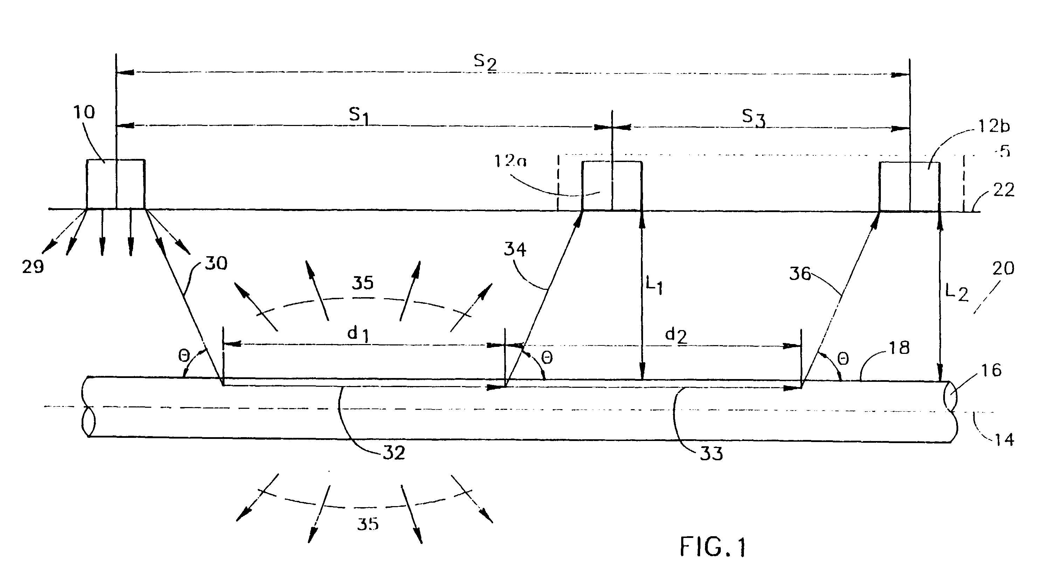

Reference is now made to Fig. 1 which schematically illustrates a method of ultrasonically measuring mechanical properties of a hard material, such as a solid 16, and apparatus which implements the method constructed and operative in accordance with the present invention. The apparatus comprises an ultrasonic transmitter 10, such as an ultrasonic transducer made of piezoelectric ceramics, typically capable of transmitting ultrasonic signals at frequencies in the range of 20 KHz to 10 MHz, and a receiver unit 5 for receiving the transmitted signals. Unit 5 typically comprises at least two ultrasonic receivers 12 such as ultrasonic transducers made of piezoelectric ceramics.

The unit 5 and transmitter 10 are placed generally in the direction of a long axis 14 of solid 16 and preferably parallel to a surface 18 of solid 16. Solid 16 is typically a metal whose mechanical properties, such as Young's Modulus (E), density (rho) and Poisson's ratio ($\sigma$), are to be measured. It can also be skeletal bone.

Along surface 18 of the solid 16 is typically an interposed medium 20, such as a gel or water for a metal solid 16 or such as soft tissue typically surrounding skeletal bone. The transmitter 10 and receivers 12 are typically placed on a top surface 22 of the interposed medium 20 wherein the receivers 12 are located at distances $s_1$ and $s_2$ from transmitter 10. In accordance with the present invention, thicknesses $L_1$ and $L_2$ of the interposed medium 20 under each of receivers 12 are preferably generally equivalent.

When the interposed medium 20 is not utilized, the transmitter 10 and receivers 12 are placed directly on surface 18 of solid 16 (this embodiment is, however, not covered by the claims).

The transmitter 10 typically transmits an ultrasonic wave 29 which propagates through the interposed medium 20 as a longitudinal wave until it reaches surface 18. A portion of wave 29, marked by arrow 30, reaches surface 18 at an angle $\Theta$ to surface 18 where $\Theta$ is the Brewster Angle. As is known in the art, the wave 29 generates in solid 16 three waves, the longitudinal, transverse and surface waves, schematically marked by arrow 32. As is known in the art, if the hard material is not a solid 16, only the longitudinal wave will be propagated.

The three waves propagate through solid 16 and generate longitudinal waves 35 in interposed medium 20 to be received by receivers 12.

Portions of waves 35, marked by arrows 34 and 36, propagate through interposed medium 20 at angle $\Theta$ with respect to surface 18. As is known in the art, the path marked by arrows 30, 32 and 34 is the shortest path an ultrasonic wave can take to reach a first one of receivers 12, marked 12a. The shortest path to reach a second one of receivers 12, marked 12b, is marked by arrows 30, 32, 33 and 36. Thus, a wave propagating along the paths marked by arrows 30, 32, 34 or 30, 32, 33 and 36 will be received first; other waves propagating along other paths will be received later.

The Brewster angle $\Theta$ is calculated as follows:

$$\Theta = \arccos(V_L'/V_L) \tag{5}$$

where $V_L'$ is the velocity of a longitudinal wave in interposed medium 20 and $V_L$ is the velocity of a longitudinal wave in hard material 16. For soft tissue, $V_L'$ is on average 1540 m/s and for bone, $V_L$ is on average 3500 m/s, producing a Brewster angle $\Theta$ of roughly 65°.

The first ultrasonic wave to reach receiver 12a is a longitudinal wave which leaves solid 16 at the Brewster Angle $\Theta$ after traveling through solid 16 for a distance $d_1$. The first ultrasonic wave to reach receiver 12b is a longitudinal wave which travels through solid 16 for a distance $d_1 + d_2$.

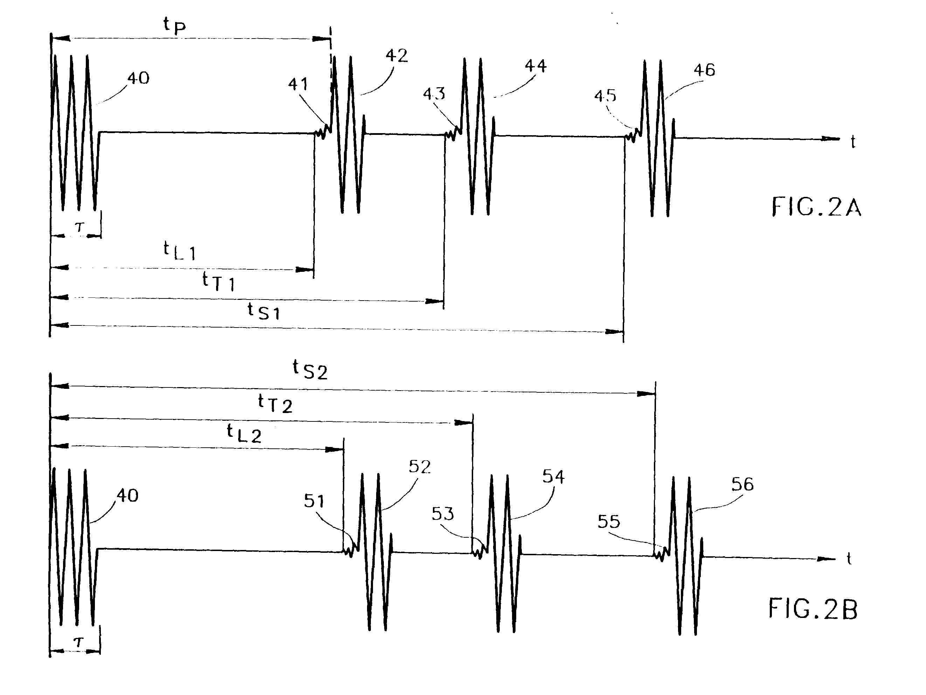

It will be appreciated that the three waves do not reach a receiver 12 at the same time due to their different velocities. This is illustrated in Figs. 2A and 2B, to which reference is now briefly made. Fig 2A illustrates a signal 40, representing the ultrasonic wave transmitted by transmitter 10, and signals 42, 44 and 46 which are received by receiver 12a and represent the longitudinal, transverse and surface waves, respectively.

Signal 40 is produced for a time tau, where tau is typically fairly short, so as to enable a separation between the longitudinal, transverse and surface waves. For example, 5 cycles of a 1MHz signal will produce a tau of 5 microseconds.

Signals 42, 44 and 46 comprise low energy portions 41, 43 and 45, respectively, corresponding to the first waves to reach receivers 12 which typically have much less energy compared to the later signals. In accordance with the present invention and in contrast to the prior art, propagation times $t_{L1}$, $t_{T1}$ and $t_{S1}$ are measured from the initiation of signal 40 (i.e. the beginning of transmission of ultrasonic wave 29) to the moment the first waves reach receivers 12. The prior art measures a propagation time $t_p$ measured from the initiation of signal 40 to the moment signal 42 reaches a significant amplitude. Propagation time $t_p$ measures the time of propagation of a reflected signal, rather than one propagated through solid 16.

As is known in the art, the larger the distance between the transmitter 10 and a receiver 12, the longer are the times $t_{L1}$, $t_{T1}$, $t_{S1}$ and the more separated are the signals 42, 44 and 46. The size of the distance $d_1$ necessary to produce a separation among the signals 42, 44 and 46 depends on the mechanical properties of the solid 16 and for bone are typically at least 5 cm.

It will be appreciated that the minimum value for $s_1$ depends on the thicknesses $L_1$ and $L_2$; the bigger $L_1$ and $L_2$ are, the bigger $s_1$ has to be to ensure that $d_1$ is greater than 0. In other words, there has to be some propagation through the solid 16. It will also be appreciated that $d_2$ has to be large enough to achieve accurate measurements and small enough to ensure that the signals 52, 54 and 56 have not overly decreased in power due to absorption of the signal energy by solid 16. Typically, $d_2$ is a few cm.

Fig. 2B is similar to Fig. 2A and illustrates signals 52, 54 and 56 received by receiver 12b. Signals 52, 54 and 56 respectively comprise low energy portions 51, 53 and 55. It will be appreciated that each of low energy portions 51, 53 and 55 are received slightly later than each of low energy portions 41, 43 and 45, respectively. The propagation times are marked $t_{L2}$, $t_{T2}$ and $t_{S2}$.

The velocity of propagation of each of the longitudinal, transverse and surface waves can be calculated by taking a known distance, such as the distance from receiver 12a and 12b, $s_3 = s_2 - s_1$, and dividing by the propagation time through that distance. The propagation time through $s_3$ is calculated for the longitudinal wave as follows.

The propagation time $t_{L1}$ is a combination of propagation times $t_{30}$, $t_{32}$ and $t_{34}$ for the waves marked by arrows 30, 32 and 34, respectively, and the propagation time $t_{L2}$ is a combination of propagation times $t_{30}$, $t_{32}$, $t_{33}$ and $t_{36}$ for the waves marked by arrows 30, 32, 33 and 36, respectively. Written mathematically, this becomes equations 6a and 6b.

$$t_{L1} = t_{30} + t_{32} + t_{34} \tag{6a}$$

$$t_{L2} = t_{30} + t_{32} + t_{33} + t_{36} \tag{6b}$$

If the thicknesses $L_1$ and $L_2$ of the interposed medium 20 under receivers 12 are generally equivalent and because the angle to both receivers 12 is $\Theta$, the propagation time through the interposed medium 20 to the receivers 12 will be equivalent, or

$$t_{34} = t_{36} \tag{7}$$

Taking the difference between equations 6b and 6a and including equation 7 produces

$$t_{L2} - t_{L1} = t_{33} \tag{8}$$

Simple geometry states that if the thicknesses $L_1$ and $L_2$ are generally equivalent and since the angle of propagation of the waves marked by arrows 34 and 36 is equivalent, the distances $d_2$ and $s_3$ will be generally equivalent. Therefore,

$$V_L = s_3/(t_{L2} - t_{L1}) \tag{9}$$

Similarly,

$$V_T = s_3/(t_{T2} - t_{T1}) \tag{10}$$

$$V_S = s_3/(t_{S1} - t_{S1}) \tag{11}$$

Thus, equations 1 - 2 can be solved to produce the Poisson's ratio $\sigma$ and a ratio E/rho as follows.

$$\sigma = \frac{B - 2}{2(B - 1)} \tag{12}$$

$$\frac{E}{rho} = \frac{V_L^2 \ (3B - 4)}{B \ (B-1)} \tag{13}$$

or

$$\frac{E}{rho} = 2V_T^2 \left[ 1 + \frac{B - 2}{2 \ (B-1)} \right] \tag{14}$$

where

$$B = (V_L/V_T)^2 \tag{15}$$

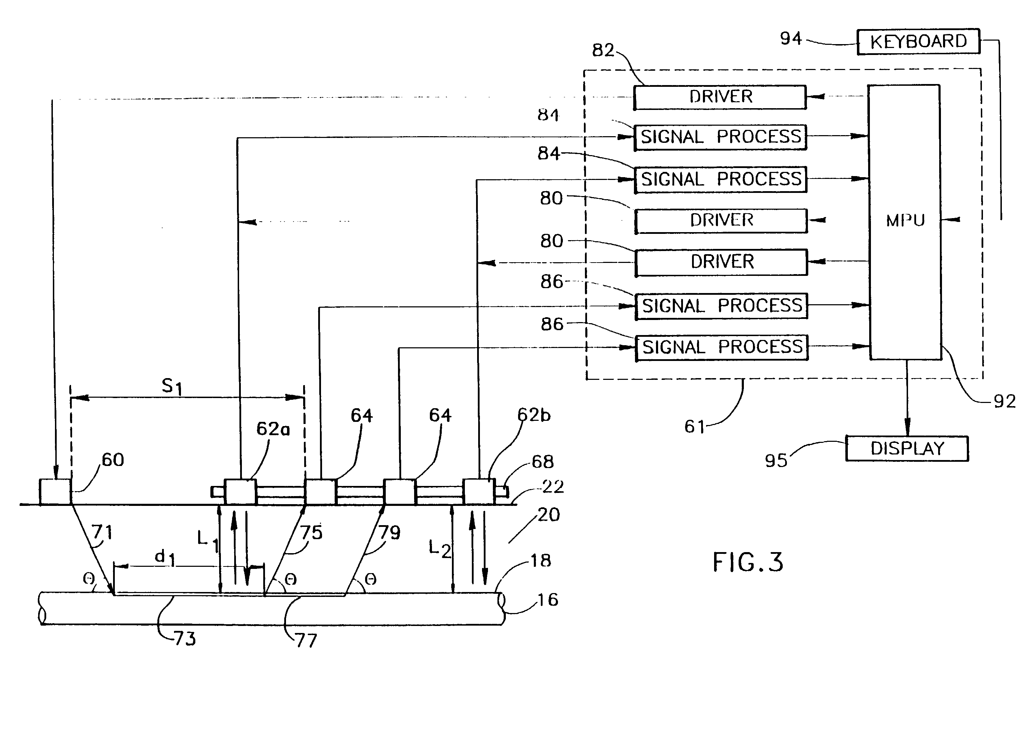

Reference is now made to Fig. 3 which illustrates apparatus for in vivo measuring of the mechanical properties of a solid, such as bone, which is surrounded by an interposed medium, such as soft tissue, of unknown and non-constant thickness, constructed and operative in accordance with a preferred embodiment of the present invention.

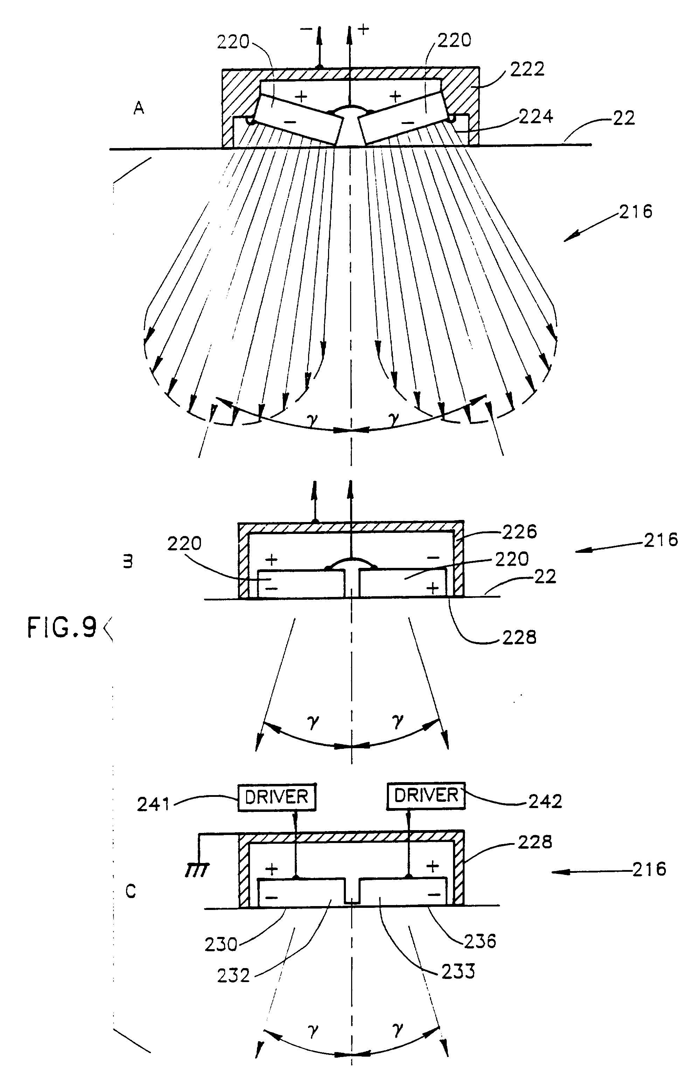

The apparatus for in vivo measuring typically comprises an ultrasonic transmitter 60, typically transmitting in the range 20 KHz - 10 MHz, for transmitting an ultrasonic wave 71, at least two ultrasonic transmitter-receivers 62 transmitting typically in the range 20 KHz - 10 MHz, for estimating the thickness of the interposed medium 20 surrounding a solid 16, a plurality of ultrasonic receivers 64 for receiving the ultrasonic signal transmitted by the transmitter 60 and a processing unit 61. Preferred embodiments for transmitter 60 and receivers 64 are shown in Figs. 9A - 9C.

The two transmitter-receivers 62 and the receivers 64 are typically combined in a unit located such that there is a distance $s_1$ between the transmitter 60 and a first receiver 64. Typically, the receivers 64 are located between the transmitter-receivers 62. Thus, if the thickness of the interposed medium 20 surrounding the solid 16 is generally equivalent under the transmitter-receivers 62, it is equivalent under the plurality of receivers 64.

In accordance with a preferred embodiment of the present invention, the two transmitter-receivers 62 are operative to transmit an ultrasonic wave in a direction generally perpendicular to the surface 22 of the interposed medium 20 and to receive the reflection of the transmitted wave from surface 18. The transmitter-receivers 62 and the receivers 64 are pressed into the interposed medium 20 so as to change their locations with respect to the solid 16 until the received signals have propagated in the interposed medium 20 for the same length of time.

Additionally, transmitter-receivers 62 are operative to estimate the thickness $L_1$. The estimate of $L_1$ is useful in estimating the distance $s_1$, useful in defining the placement of transmitter 60. Through geometric considerations, $s_1$ is approximated as follows:

$$s_1 \sim d_1 + 2L_1/\tan\Theta \qquad\qquad (16)$$

Since, as mentioned hereinabove, the distance $d_1$ must be greater than 0, $s_1$ is calculated to be larger then $2L_1/\tan\Theta$, typically by 30 - 40%. Alternatively, $s_1$ can be a fixed distance from the receivers 64.

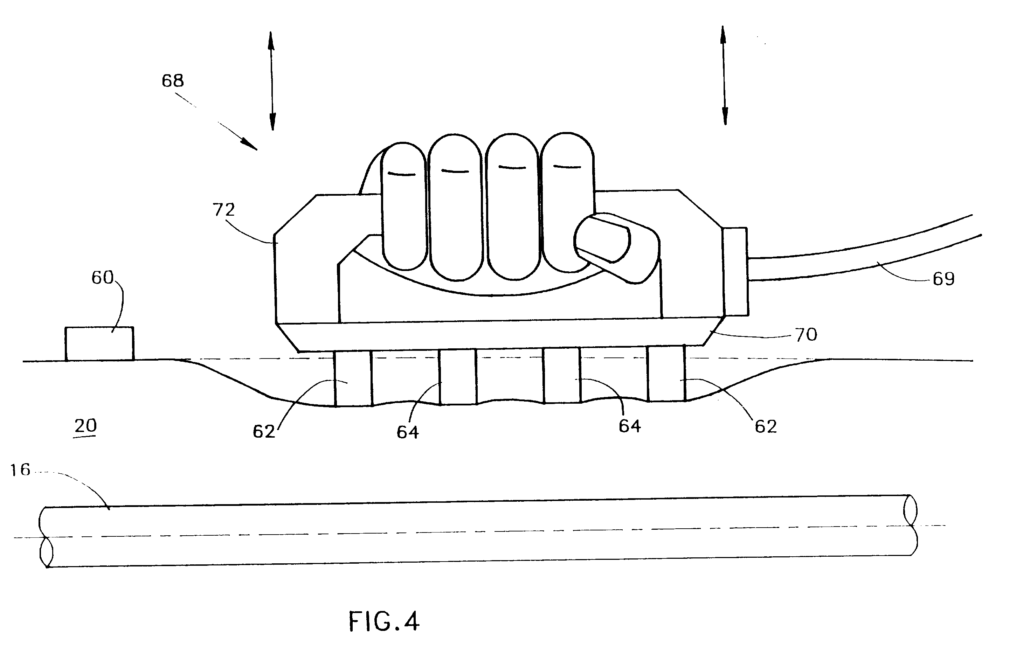

To effect the change in location of the transmitter-receivers 62 and the receivers 64, the elements 62 and 64 are combined together in a single rockable unit 68 as shown in Fig. 4 to which reference is now briefly made. The elements 62 and 64 are held together in a unit 70 to which is attached a rocking unit 72, such as a handle, to rockingly press the elements 62 and 64 into the interposed medium 20. By "rockingly press" it is meant that the rocking unit 72 is slowly rocked from side to side while pressing into the interposed medium 20. Attached to the rockable unit 68 are cables 69 for connecting the rockable unit 68 to the processing unit 61.

It will be appreciated that rockable unit 68 can be any suitable unit, whether manually or automatically operated.

It will also be appreciated that rockable unit 68 can be slidingly moved along surface 22 while measurements are performed, as described hereinbelow. Accordingly, measurements along the solid 16 can be taken, giving an operator an indication of locations of changes or discontinuities in the quality of the material of solid 16.

While the rockable unit 68 is being rocked, the transmitter-receivers 62 continually transmit and receive ultrasonic waves. The received signals are sent to the processing unit 61 which continually measures receipt times of the two received signals, where a receipt time is the time to measure the thicknesses $L_1$ and $L_2$. When the difference between the receipt times of the two received signals is generally zero, indicating that the thickness of the interposed medium 20 between the two transmitter-receivers 60 and the solid 16 is generally equivalent, the processing unit 61 causes the transmitter 60 to transmit an ultrasonic wave, marked by arrow 71.

Receivers 64 receive the ultrasonic wave after it has passed through the interposed medium 20 and the solid 16, as shown by arrows 73, 75, 77 and 79. Processing unit 61 receives the received signals, calculates at least $t_{L_1}$ and $t_{L_2}$, and from them, calculates $V_L$ which is correlated to the mechanical properties of bone, as defined in Equation 4b. Additionally, processing unit 61 can calculate the ratio E/rho and a from equations 12 - 15.

It will be appreciated that the transmission and reception of the ultrasonic signals occurs within typically 200 microseconds so that any changes due to rocking of the rockable unit 70 have generally no effect on the thickness of the interposed medium 20 under the receivers 64 during the measurement.

Reference is made back to Fig. 3. The processing unit 61 comprises drivers 80 and 82 for driving ultrasonic transmitter-receivers 62 and transmitter 60, respectively, and signal processor units 84 and 86 for processing the received signals from transmitter-receivers 62 and from receivers 64, respectively.

Processing unit 61 additionally comprises a Main Processing Unit (MPU) 92, such as a microcontroller, for controlling the apparatus through sending driving signals to drivers 80 and 82 and receiving and processing signals from signal processor units 84 and 86. MPU 92 additionally interfaces with an operator of the apparatus of the present invention through a keyboard 94 and a display 95.

If $s_1$ is not fixed, at the beginning of a measurement, it is approximated as follows. Rockable unit 68 is rockingly pressed into interposed medium 20 while MPU 92 pulses transmitter-receivers 62 and receives signals from them via signal processor units 84 using the larger threshold level $T_2$. When delta_t, the time difference between the receipt times of the received signals, is generally zero, MPU 92 calculates the time $t_{tr}$ between the transmission and receipt of each signal sent by and reflected to each transmitter-receiver 62. By multiplying $t_{tr}$ by the known average velocity of sound in soft tissue, MPU 92 calculates $L_1$ which is then used in equation 17 for defining the distance $s_1$.

To take a measurement, the rockable unit 68 is again rockingly pressed into interposed medium 20 while MPU 92 alternates, at a high frequency, between pulsing one transmitter-receiver, marked 62a, and the other transmitter-receiver, marked 62b. It receives the processed signals from signal processor units 84 and calculates a time difference delta_t between the receipt times of the received signals from transmitter-receivers 62. When delta_t is generally zero, MPU 92 pulses driver 82 to produce the ultrasonic wave marked by arrow 71 and stops pulsing drivers 80.

The ultrasonic wave produces ultrasonic waves, marked by arrows 75 and 79, which are received by receivers 64. The received signal is processed by signal processors 86 and the output is sent to MPU 92 for evaluation of at least the time difference $t_{L2}$ - $t_{L1}$ and for calculation of at least the velocity $V_L$, in accordance with equation 9.

It will be appreciated that the apparatus of the present invention is operative to calculate $V_S$ and $V_T$ also, utilizing the method outlined hereinabove with respect to Figs. 1 and 2. From the calculation of $V_L$, $V_S$ and $V_T$, the ratio E/rho and $\sigma$ can be calculated. It will be noted, however, that the calculation of $V_L$ is necessary for comparing the results of the apparatus of the present invention with results of other prior art devices.

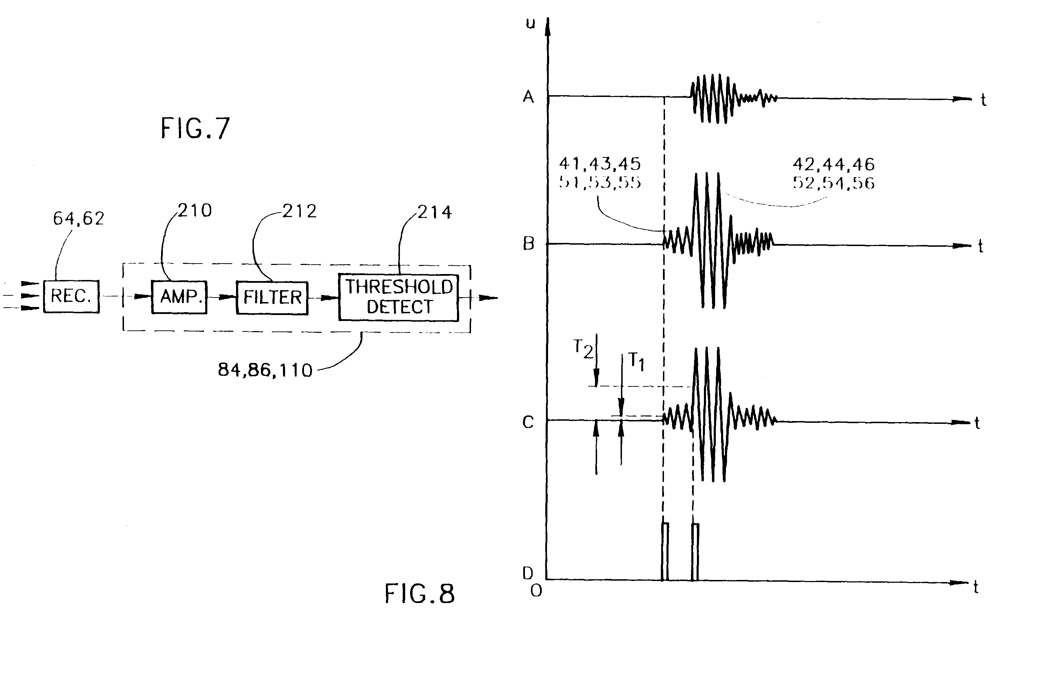

Reference is now made to Figs. 7 and 8A - 8D which respectively illustrate the elements of signal processor unit 84 or 86 and their operation.

In order to measure the moment the first waves reach receivers 64, signal processor unit 84 or 86 comprises an automatic gain control amplifier 210 of high sensitivity and low noise/signal ratio for exponentially amplifying signals 42 and 52 so as to measure the initiation of portions 41 and 51 and to compensate for the typical exponential attenuation of ultrasonic signals. The output of the amplifier 210 is shown in Fig. 8B for an input signal 42 of Fig. 8A where signal 42 comprises a low energy portion 41.

Signal processor unit 84 or 86 additionally comprises a band pass filter 212 for filtering out all signals not in the range of frequencies transmitted by transmitter 60, thereby to reduce noise in the received signal produced by the amplification or otherwise. Its output is shown in Fig. 8C for the input signal of Fig. 8B.

Furthermore, each signal processor unit 84 and 86 comprises a threshold detector 214 for identifying the initiation of either signals 42 and 52 or portions 41 and 51, respectively. Thus, threshold levels $T_1$ and $T_2$, shown in Fig. 8C, are defined. $T_1$ identifies the portions 41 and 51 and $T_2$ identifies the signals 42 and 52. The output of threshold detector 214 is shown in Fig. 8D for an input signal of Fig. 8C.

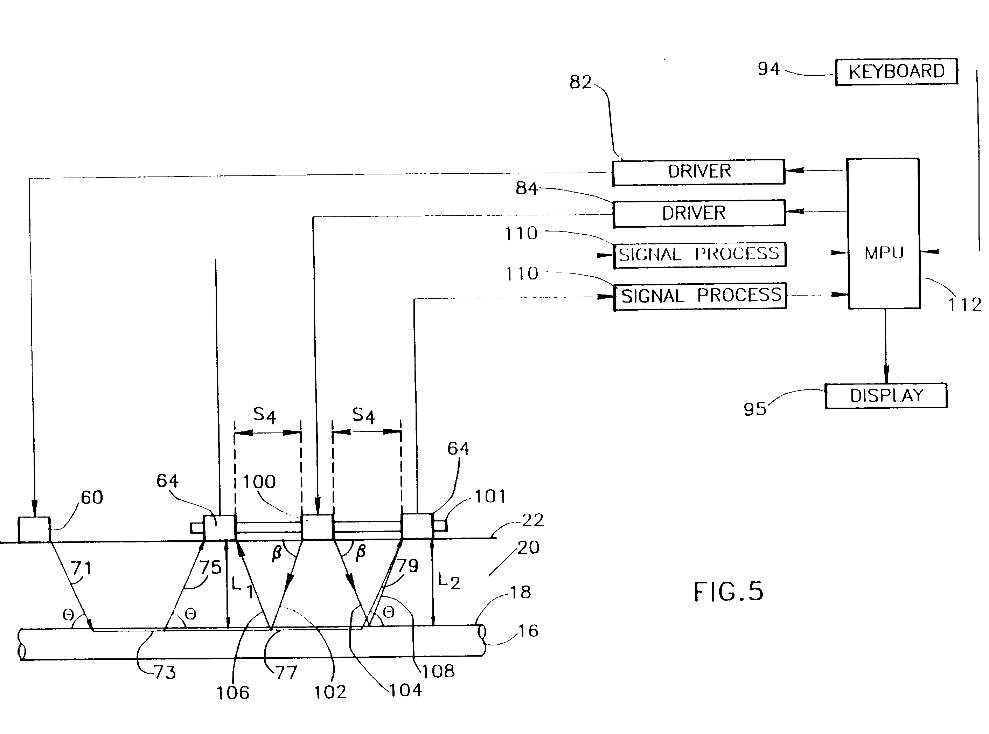

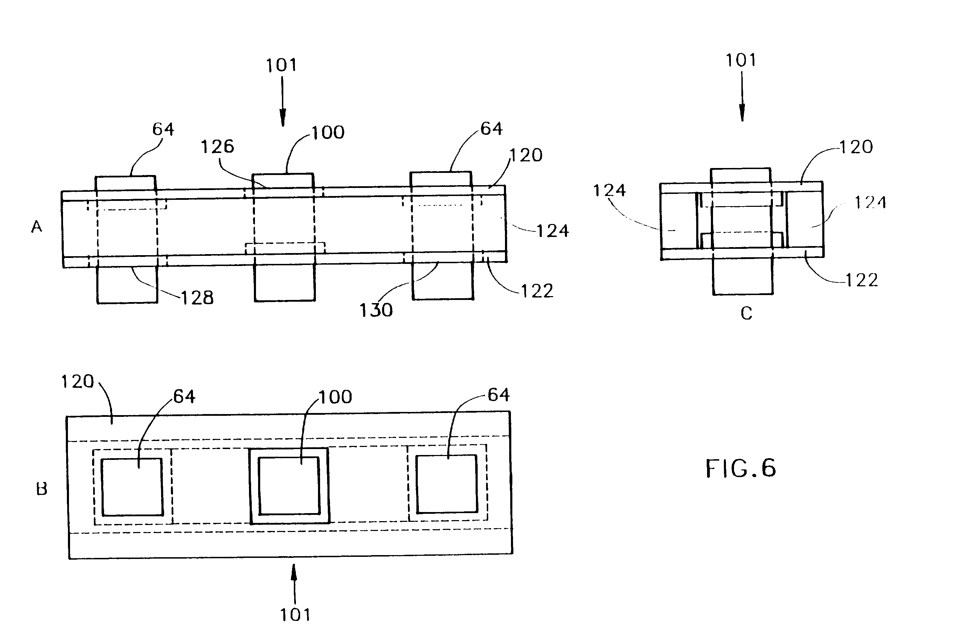

Reference is now made to Fig. 5 which illustrates an alternative embodiment of the apparatus of Fig. 3. In this embodiment, the two transmitter-receivers 62 are replaced by a single transmitter 100, described in more detail hereinbelow with reference to Figs. 9A - 9C, located exactly equidistant between an even plurality of receivers 64. Transmitter 100 and at least two receivers 64 are connected together via an acoustically insulated unit 101. Unit 101 is shown in detail in Figs. 6A - 6C.

Transmitter 100 transmits at least two ultrasonic waves at a general direction having a non-zero angle gamma (Figs. 9A - 9D) with respect to a perpendicular axis of surface 22 (not shown). A portion of the two waves, marked 102 and 104 in Fig. 5 and having an angle $\beta$ as shown, are reflected from solid 16 to receivers 64 as waves 106 and 108. The angle $\beta$ is unknown and depends on the thicknesses $L_1$ and $L_2$.

Receivers 64 receive reflections 106 and 108, from the surface 18, and send the received signal to signal processors 110, similar to signal processors 84 and 86 of the embodiment of Fig. 3, for processing with both threshold level $T_1$ and $T_2$. The processed signal is then sent to a MPU 112, similar to MPU 92 of the embodiment of Fig. 3.

If $s_1$ is not fixed, at the beginning of a measurement, it is approximated as follows. Unit 101 is rockingly pressed into interposed medium 20 while MPU 112 pulses transmitter 100 and receives signals from receivers 64 via signal processor units 110 using the larger threshold level $T_2$. When delta_t, the time difference between the receipt times of the received signals, is generally zero, MPU 112 calculates the time $t_{tr}$, similar to time $t_p$ of Fig. 2A, between the transmission and receipt of each signal sent by transmitter 100 and received by receivers 64.

Once $t_{tr}$ is calculated, the thickness $L_1$ is approximated as follows:

$$L_1 = \sqrt{[(V_L{'}*t_{tr}/2)^2 - (s_4/2)^2]} \qquad (17)$$

where $s_4$ is the distance between transmitter 100 and a receiver 64. Since, as mentioned hereinabove, the distance $d_1$ must be greater than 0, $s_1$ is calculated, in accordance with equation 16, to be larger then $2L_1/\tan\Theta$, typically by 30 - 40%.

During a measurement, unit 101 is again rockingly pressed into interposed medium 20 while MPU 112 pulses transmitter 100 and receives signals from receivers 64 via signal processor units 110, using the larger threshold level $T_2$. When delta_t is generally zero, MPU 112 pulses driver 82 to produce the ultrasonic wave marked by arrow 71 and stops pulsing driver 84.

The ultrasonic wave produces ultrasonic waves, marked by arrows 75 and 79, which are received by receivers 64. The received signal is processed by signal processors 110, using threshold level $T_1$, and the output is sent to MPU 112 for evaluation of at least the time difference $t_{L2} - t_{L1}$ and for calculation of at least the velocity $V_L$, in accordance with equation 9.

Reference is now made to Figs. 6A - 6C which are side, top and end view illustrations of the acoustically insulated unit 101. The transducers 64 and 100 of Fig. 5 are enclosed in two rigid frames 120 and 122, typically of metal, between which is an acoustically absorbent material 124, such as elastic rubber. Receivers 64 are attached to frame 120 and transmitter 100 is attached to frame 122, wherein frame 120 has a hole 126 in which transmitter 120 sits and frame 122 has holes 128 and 130 in which sit receivers 64.

The material 124 is operative to absorb ultrasonic waves which are transmitted by transmitter 100 such that receivers 64 do not receive such waves before receipt of the ultrasonic waves propagated through the solid 16. Frames 120 and 122 are operative to provide a firm shape to material 124. Each frame only holds one type of ultrasonic transducer; for example, frame 120 holds only receivers 64.

Unit 101 is necessary to ensure that the ultrasonic wave transmitted by transmitter 100 is received by receivers 64 only after having propagated through solid 16. Without the acoustic insulation provided by unit 101, it is possible that the first signals received by receivers 64 are those from wave which propagated through frames 120 and 122.

Reference is now made to Figs. 9A - 9C which illustrate preferred embodiments of an ultrasonic transceiver 216 operative as transmitters 60 and 100 and receivers 64. Transceiver 216 is operative to transmit an ultrasonic wave 71 at an angle gamma and to receive energy which arrives generally in that direction thereby increasing the amount of energy available in low energy portions 41 and 51.

Ultrasonic transceiver 216 is configured to produce ultrasonic waves in two directions, marked by angles gamma, and generally no wave in the direction perpendicular to the surface 22 on which transceiver 216 stands. Typically, gamma = 90 - $\Theta$ .

In Fig. 9A, transceiver 216 comprises two standard ultrasonic transducers 220 having positive and negative terminals, denoted by + and - signs, respectively, whose positive terminals are connected together. Transducers 220 are housed in a housing 222 comprising two angled portions 224 on which sit transducers 220. The angled portions 224 are typically comprised of an acoustically transmissive material, such as gel. In this embodiment, the negative terminals are connected to ground and the positive terminals are connected to a driver.

A second embodiment of transceiver 216 is shown in Fig. 9B. As in the previous embodiment, transceiver 216 comprises two standard transducers 220 enclosed in a housing, labeled 226. In this embodiment, the positive terminal of one transducer 220 is connected to the negative terminal of the other, and vice versa. As is known in the art, this produces waves having opposite phases; thus, only the waves generally at an angle gamma are produced. One positive-negative terminal pair is placed on an electrode 228 which is typically placed along surface 22. The other positive-negative terminal pair, which is connected to a driver, is located inside housing 226. The housing 226 is connected to ground.

Fig. 9C illustrates a third embodiment of transceiver 216 which is housed in a housing 228 and is comprised of a single ultrasonic transducer 230 which is at least partially cut into two sections 232 and 233. The negative terminals of sections 232 and 233 are typically attached to an electrode 236 and the positive terminals of sections 232 and 233 are connected to opposite polarity drivers 240 and 241 such that section 232 receives a positive signal at the same time that section 233 receives a negative signal, and vice versa.

It will be appreciated that what has been described herein is merely illustrative of the method and apparatus of the present invention. Other methods of achieving the same goal are included in the present invention. For example, an alternative method for defining $s_1$ is as follows.

In Fig. 2A, signal 42 is typically produced by ultrasonic waves reflecting from the surface 18 of solid 16 and portion 41 is produced by waves propagating a distance $d_1$ through the solid 16. Thus, if there is a difference between the receipt time $t_{L1}$ of portion 41 and the receipt time $t_p$ of signal 42 then $d_1$ is greater than 0 as required. Measurement of $t_{L1}$ and $t_p$ merely requires that signal processors 86 and 100 have the two threshold levels $T_1$ and $T_2$.

It will be appreciated that the apparatus of the present invention is operative for thicknesses of an interposing medium 20 which are not equivalent, although the results are less accurate. Referring once again to Fig. 1, if the receipt times $t_{L1}$ and $t_{L2}$ differ by the measured value delta_t, then equations 6 - 8 are rewritten as follows into equations 18 - 20:

$$t_{L1} = t_{30} + t_{32} + t_{34} \qquad\qquad (18a)$$

$$t_{L2} = t_{30} + t_{32} + t_{33} + t_{36} \tag{18b}$$

where

$$t_{34} = t_{36} + \text{delta\_t} \tag{19}$$

and delta_t might be positive or negative . Thus,

$$t_{L2} - t_{L1} = t_{33} + \text{delta\_t} \tag{20}$$

Since, due to geometric considerations, if $L_1$ is not equivalent to $L_2$ then $d_2$ is not necessarily equivalent to $s_3$. However,

$$L_1 = L_2 + \text{delta\_L} = L_2 + (\text{delta\_t})(V_L') \tag{21}$$

and therefore,

$$d_2 = \sqrt{(s_3{}^2 - (\text{delta\_L})^2} \tag{22}$$

and

$$V_L = d_2/(t_{L2} - t_{L1}) \tag{23}$$

It will further be appreciated that the apparatus of the present invention is operative without an interposing medium 20 (this embodiment is, however, not covered by the claims). For such a situation, unit 5 (Fig. 1) comprises only one receiver 12a and the equations 9 - 11 are:

$$V_L = s_1/t_{L1} \tag{24}$$

$$V_T = s_1/t_{T1} \tag{25}$$

$$V_S = s_1/t_{S1} \tag{26}$$

Further alternative methods and apparatus for implementing the present invention are described hereinbelow.

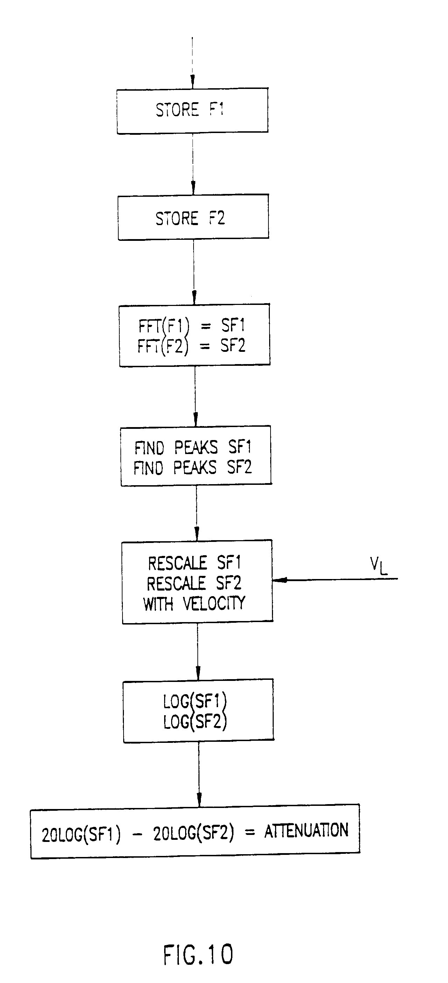

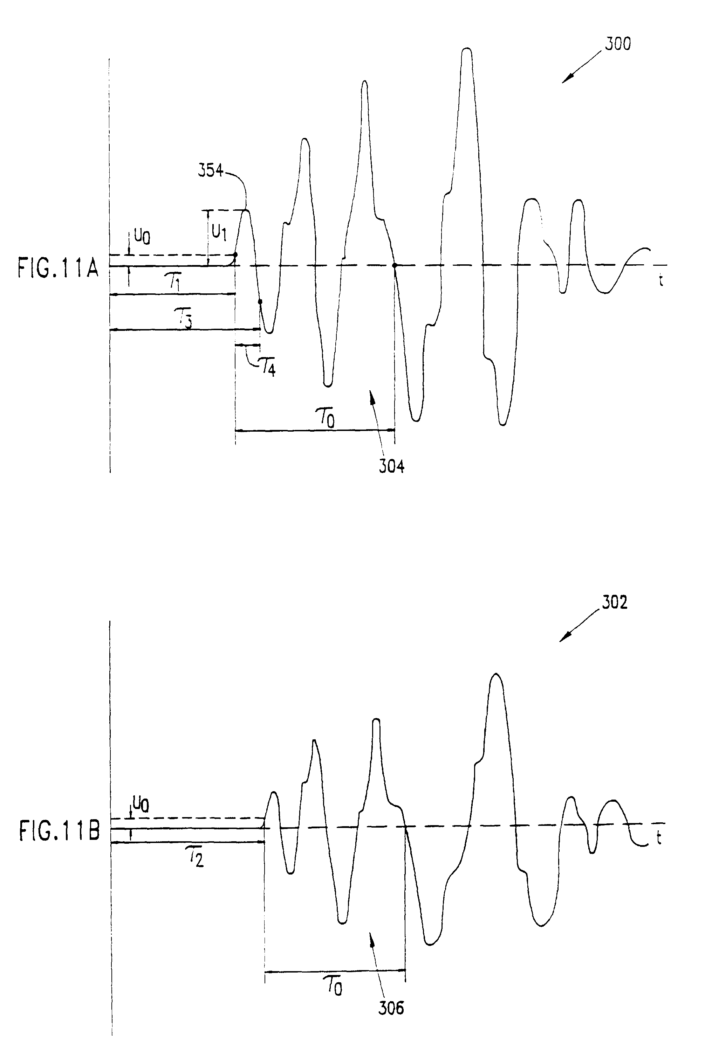

Reference is now made to Fig. 10 which illustrates, in flow chart format, an additional method of signal processing performed by either of the MPUs 92 and 112 and to Figs. 11 - 13 which are useful in understanding the method. The method will be described in conjunction with Fig. 3, it being understood that the elements of Fig. 5 can also perform the method.

The two signal processors 86 each receive a signal from receivers 64. The first signal 300 received, shown in Fig. 11A, is received at a time $\tau_1$ after the transmitter 60 provides its input signal while the second signal 302 received, shown in Fig. 11B, arrives at a time $\tau_2$.

In this method, the signal processors 86 additionally provide to the MPU 92 portions 304 and 306 of the signals 300 and 302, respectively, each typically of $\tau_o$ seconds in length. Typically, the portions 304 and 306 correspond to the initial waves which arrive at each receiver 64.

The MPU 92 or 112 stores portions 304 and 306 (denoted F1 and F2, respectively, in Fig. 10) and, when not otherwise driving the system, the MPU 92 or 112 performs a Fast Fourier Transform (FFT) on each of F1 and F2, producing signals SF1 and SF2. $\tau_o$ therefore depends on the sampling rate and the number of samples desired for use in the FFT.

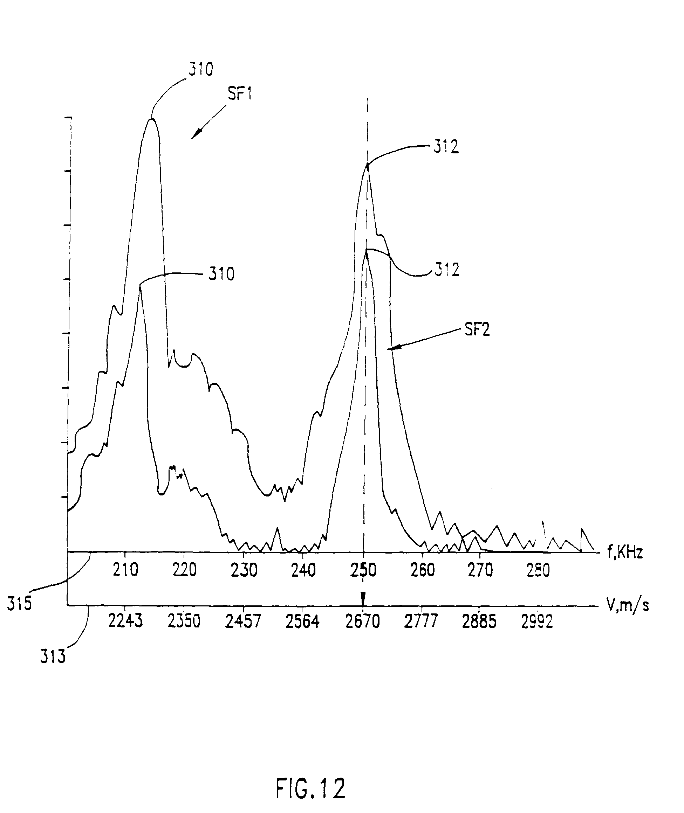

Signals SF1 and SF2 are shown in Fig. 12. It is noted that they each comprise a plurality of peaks 310 and 312 and that signal SF2 is attenuated vis-a-vis signal SF1. The peaks indicate a natural filtering frequency of each of the materials which comprise the solid, wherein the frequency corresponds to the propagation speed which is the velocity at which the ultrasonic wave transmitted by transmitter 60 travels through the solid.

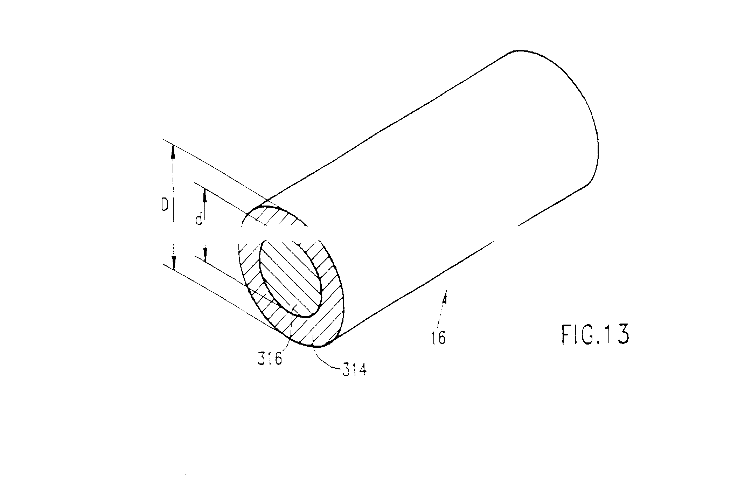

For solids 16 formed of two or more materials 314 and 316, such as is shown in Fig. 13, the signals SF1 and SF2 will comprise two or more peaks, respectively. The highest frequency peak 312 corresponds to the "fastest" material (i.e. the material through which the transmitted wave travels fastest) and its corresponding velocity is the velocity $V_L$ calculated by the MPU 92 in accordance with equation 9 hereinabove.

Because of the relationship between frequency and velocity, the horizontal axis of Fig. 12 can be rescaled to indicate velocity. The velocity scale is indicated by reference numeral 313 and the frequency scale is indicated by reference numeral 315. The values provided are appropriate for a solid 16 comprised of the following materials: epoxy and Perspex.

The attenuation between signals SF1 and SF2 occurs because signal 302 travels through a longer portion of solid 16 than does signal 300. Thus, the attenuation corresponds to the attenuation which occurs in the portion $d_2$ of solid 16.

The method shown in Fig. 10 identifies the velocity of each of the peaks and the attenuation caused by traveling through portion $d_2$.

The method identifies the peaks 310 and 312 in each of SF1 and SF2 and matches the value for the velocity $V_L$, received from equation 9, to the last peak 312. The horizontal axis can then be rescaled and from the velocity scale 313, the velocities corresponding to the other peaks 310 can be calculated. The peak 312 corresponds to the fastest material, which, for example, is material 314.

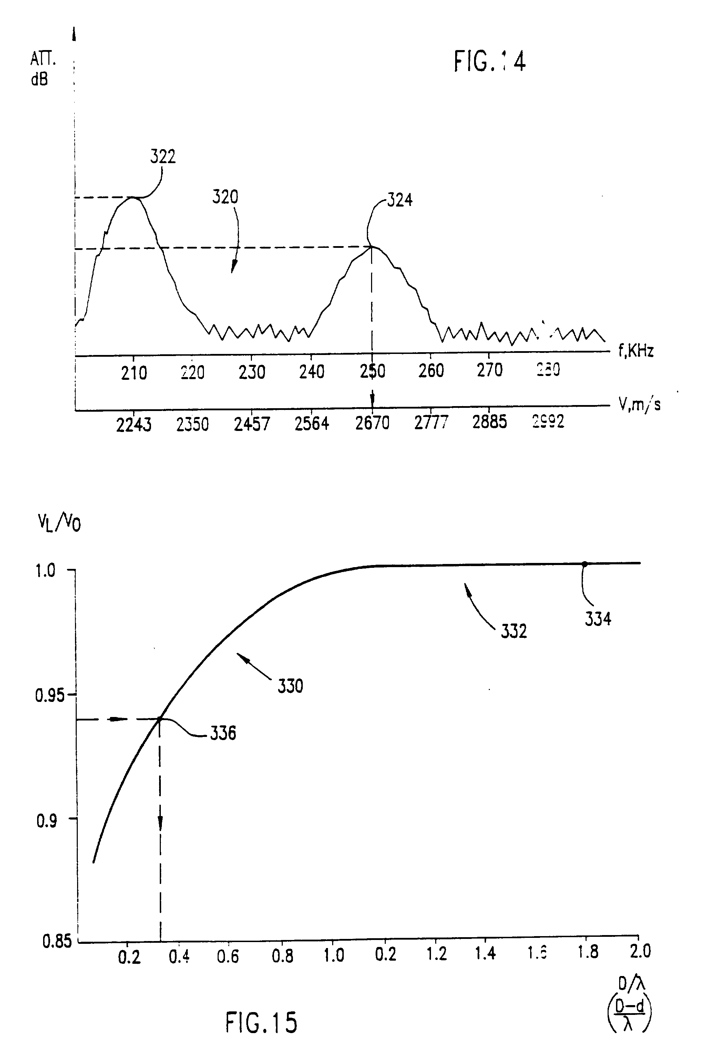

The method then calculates the logarithm (in base 10) of the amplitudes of each of signals SF1 and SF2 and subtracts log(SF2) from log(SF1) to produce a difference signal 320, shown in Fig. 14. The difference signal 320 typically comprises one or more peaks 322 and 324 corresponding to the peaks 310 and 312 of signals SF1 and SF2.

The amplitudes of the peaks 322 and 324 are the attenuation caused by the $d_2$ section of each of materials 316 and 314, respectively.

In accordance with a further embodiment of the present invention, the MPU 92 estimates the thickness of the solid 16 through utilization of an empirically-derived, non-dimensional curve of normalized velocity vs. normalized thickness, as shown in Fig. 15 to which reference is now made. A discussion of the creation of the curve in Fig. 15 is discussed in the book, Stress Waves in Solids, written by H. Kolsky, Oxford and Clarendon Press, 1953.

The precise shape of the curve varies with the type of material being measured. However, it is has been determined by the present inventors that the shape of the curve is approximately constant over the set of human bones.

The velocity $V_L$ in the curve of Fig. 15 is normalized by the velocity $V_o$ that would be achieved in an infinite solid and the thickness is normalized by the input wavelength, lambda, of the signal from the transmitter 60. It has been determined by the inventors that the curve is approximately the same whether the thickness is the thickness D (Fig. 13) of the solid 16 or it the thickness D - d (Fig. 13) of the outside cylindrical material 314.

It is noted that the curve has a region 330, for small velocity ratios and small diameter/wavelength ratios and a region 332 for diameter/wavelength ratios greater than about 1.5 which is asymptotic to 1.0.

To estimate the thickness (D - d) for a solid 16, transmitter 60 is operated twice, once with a high frequency input signal and once with a low frequency input signal. For each measurement, the signal processors 84 and the MPU 92 operate, as described hereinabove with respect to Fig. 3, to determine the received velocity.

The response to the high frequency input signal, which has a low wavelength lambda, provides a velocity datapoint 334 somewhere along the region 332 from which the velocity $V_o$ can be determined. However, the precise location of datapoint 334 is unknown since the thickness is not known.

The response to the low frequency input signal provides a velocity datapoint 336 somewhere within the region 330. Because the velocity $V_L$ is known from the measurement and the velocity $V_o$ is known from the previous measurement, the location on the curve of the datapoint 336 is known. Therefore, the ratio (D - d)/lambda can be determined. Since lambda is known from the frequency of the transmitter 60, the thickness of the solid 16, either D or (D - d), can be determined.

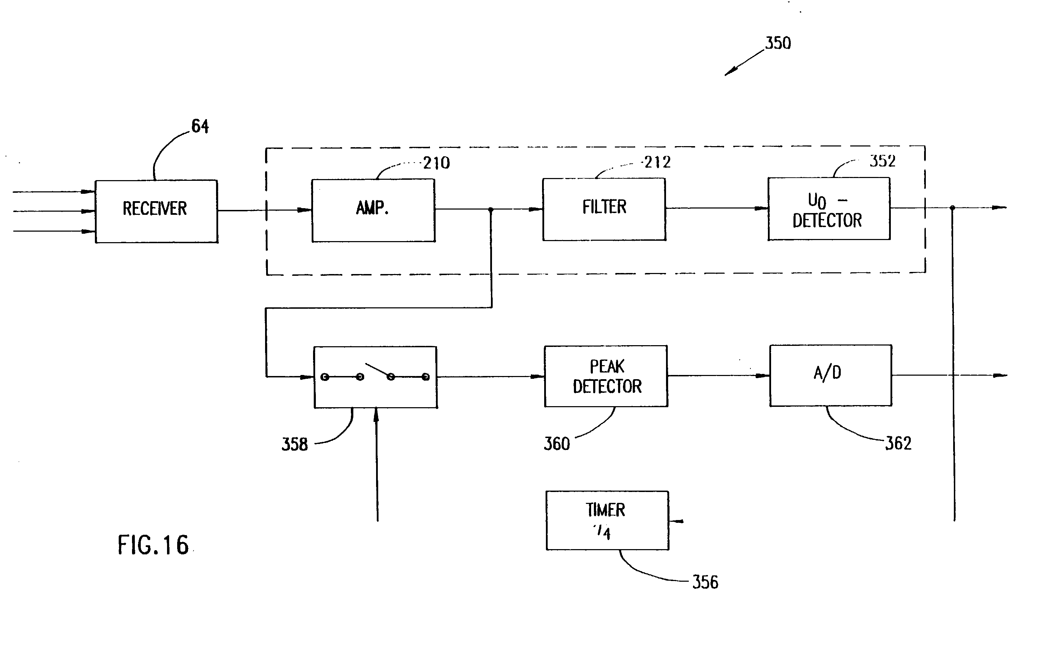

The attenuation for the faster material only can also be determined directly from the signals 300 and 302 (Figs. 11A and 11B) by anticipating the timing of the first peak in signals 300 and 302. Fig. 16, to which reference is now made, illustrates, in block diagram format, a unit 350 for determining the attenuation. Hereinbelow characteristics of signal 300 will be discussed, it being understood that both signals 300 and 302 have these characteristics.

Unit 350 detects a first threshold $U_0$, defined as a voltage above the expected noise level, in a manner similar to the apparatus shown in Fig. 7. Therefore, unit 350 typically comprises an amplifier, such as amplifier 210, a filter 212 and a $U_0$ detector 352, similar to threshold detector 214.

Since it is known that a first peak 354 follows after the voltage reaches the threshold level $U_0$, once $U_0$ detector 352 detects the voltage above $U_0$, it signals a timer 356 to open an analog switch 358 for a predetermined length of time $\tau_4$ during which the first peak 354 is expected to appear.

The analog switch 358 receives the output of the amplifier 210 and, while switch 358 is open, the output of amplifier 210 is provided to an analog peak detector 360 which then detects the amplitude $u_1$ of the peak 354.

The amplitude $u_1$ is provided to an analog-to-digital converter (ADC) 362 for conversion to a digital value.

The unit 350 is provided for the output of each receiver 64 and the value of $u_1$ for the first receiver 64, denoted A, is compared to that for the second receiver 64, denoted B. The ratio of the two values A and B, in dB, is the attenuation of the section of solid 16 of length $d_2$ (Fig. 1). In other words, the attenuation is:

$$attenuation = 20(\log_{10}A - \log_{10}B) \qquad (27)$$

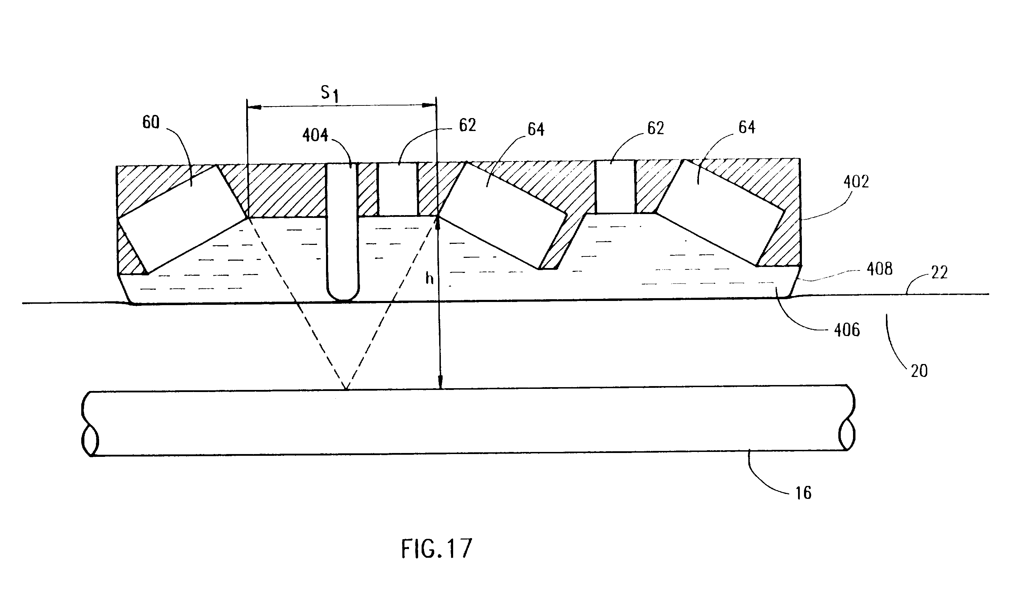

Reference is now made to Fig. 17 which illustrates the apparatus of Fig. 3 combined into a single unit 400. Elements which are similar to those in previous embodiments have similar reference numerals.

Unit 400 comprises a holding frame 402 shaped to support the transmitter 60, the transmitter-receivers 62 and the receivers 64 at desired angles, where the angles are typically the average of the expected Brewster angles for the material to be measured. For human bone, the Brewster angle varies between 22 and 30° and thus, the angle of the receivers 64 is approximately 26°.

The ultrasonic elements 60 - 64 are coupled to the surface 22 of the interposed medium 20 via a coupling material 406, such as water, oil or an acoustic gel, held in place via a flexible cover 408, such as rubber. Flexible cover 408 typically is sealingly attached to holding frame 402.

Unit 400 also comprises an acoustic barrier 404, located between the transmitter 60 and the elements 62 and 64, for ensuring that generally no cross-coupling occurs between transmitter 60 and transmitter-receivers 62. Acoustic barrier 404 can be formed of any suitable material, such as rubber, and, as shown in Fig. 17, extends through the coupling material 406 to the flexible cover 408. Because of the acoustic barrier 404, the ultrasonic waves of the transmitter 60 generally only reach the receivers 64 after traveling through the interposed medium 20.

The holding frame 402 is designed to locate the transmitter 60 a distance $s_1$ from its closest receiver 64, where $s_1$ is shown in Fig. 1. As discussed previously, $s_1$ must be large enough to ensure that $d_1$ is non-negative and small enough to ensure that the received signal is large enough to measure.

It is noted that the unit 400 is operable for interposed media 20 of a given range of thicknesses, the maximum of which is the distance H from the receivers 64 to the surface 18 of the solid 16, where $H = (s_1/2)ctg\Theta$ .

The unit 400 is typically first placed onto the surface 22 of the interposed medium 20 onto which a further coupling material (not shown) is also placed. The unit is then operated, as in previous embodiments, by rocking. The rocking will cause either or both of the coupling material 406 and the interposed medium 20 to be pressed. The coupling material ensures that the entirety of the ultrasonic elements 60 - 64 remain acoustically coupled with the interposed medium 20 during rocking.

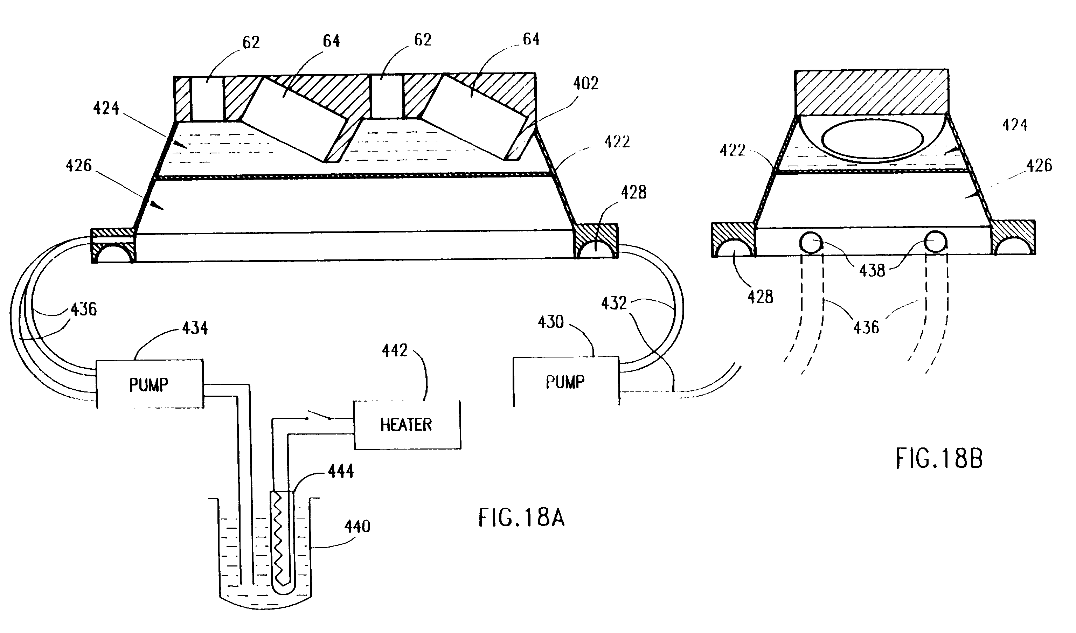

Reference is now made to Figs. 18A and 18B which respectively illustrate side and front views of an alternative embodiment of the rockable unit 68 of Fig. 4.

In this alternative embodiment, the receivers 64 and the transmitter-receivers 62 are coupled to the interposing medium 20 via a "half-bath" 420 comprised of a rubber frame 422 formed of two sections, a closed section 424 and an open section 426.

Closed section 424 encloses a coupling material, such as acoustic gel, which is permanently enclosed therein.

The portion of rubber frame 422 which forms open section 426 ends in a circular vacuum hole 428. When the air in hole 428 is removed, a suction is created by which half-bath 420 is attached to the surface 22 of interposed medium 20, thereby forming a sealed bath into which a coupling material, such as water, can be introduced.

The suction is created via a first pump 430 having inlet and outlet tubes 432 and a second pump 434 introduces the water into open section 426 via pipes 436. Pipes 436 are attached to circular holes 438 located above the vacuum hole 428 within the rubber frame 422.

The second pump 434 provides water, stored in a container 440, whose temperature can be regulated by a temperature regulator comprised of a heater 442 and a thermostat 444. The temperature regulation is typically incorporated when the present embodiment is placed on human skin and is operative to ensure that the water is at a temperature comfortable to humans.

As in previous embodiments, the apparatus shown in Figs. 18A and 18B is rocked to find the balance point.

It will be appreciated by persons skilled in the art that the present invention is not limited to what has been particularly shown and described hereinabove. Rather the scope of the present invention is defined only by the claims which follow:

Claims

1. Apparatus for determining, through an interposed medium (20), the mechanical properties of a solid (16) having a surface (18), the apparatus comprising:

   ultrasonic transmission means (10,60) located in a first location for transmitting ultrasonic waves through said interposed medium and through said solid and generally parallel to said surface;
   at least one ultrasonic receiver (12a,12b,64) unit located in each of second and third locations for receiving said ultrasonic waves, wherein said first, second and third locations are colinear along said surface;
   means for locating (61) said at least one receiver unit such that a first receipt time of an ultrasonic wave from said surface to said at least one ultrasonic receiver unit at said second location is generally equivalent to a second receipt time from said surface to said at least one ultrasonic receiver unit at said third location; and
   means for calculating (61) said mechanical properties from ultrasonic waves transmitted by said ultrasonic transmission means once said first and second receipt times are generally equivalent.

2. Apparatus according to claim 1 and wherein said means for locating adjusts the locations of said at least one receiver unit such that said first and second receipt times are generally equivalent.

3. Apparatus according to claim 2 and wherein said means for locating includes means for determining said first and said second receipt times.

4. Apparatus according to claim 3 and wherein said means for determining includes means for pulsing said transmission means thereby to transmit ultrasonic waves when said first and second receipt times are generally identical.

5. Apparatus according to claim 1 and wherein said solid is in vivo bone.

6. Apparatus according to claim 1 and wherein said solid is selected from the group of metal, plastics and wood.

7. Apparatus according to claim 1 and wherein the velocity of said ultrasonic waves through said interposed medium is less than the velocity of said ultrasonic waves through said solid.

8. Apparatus according to claim 1 and wherein said at least one ultrasonic receiver unit is placed onto a surface of said interposed medium generally without any coupling medium.

9. Apparatus according to any of the preceding claims, and wherein: said at least one ultrasonic receiver unit comprises

   at least first and second ultrasonic receiver units; and
   said means for locating said ultrasonic receiver units includes a rocker unit for rockingly pressing said ultrasonic receiver units into said interposed medium such that a first receipt time of said ultrasonic wave from said surface to said first ultrasonic receiver unit is generally equivalent to a second receipt time from said surface to said second ultrasonic receiver unit.

10. Apparatus according to claim 9 and wherein said rocker unit comprises an acoustically insulating unit.

11. Apparatus according to claim 1 wherein said ultrasonic transmission means includes means for transmitting said ultrasonic wave within a wide angle and wherein said ultrasonic waves generate longitudinal, surface and transverse waves in said solid.

12. Apparatus according to claim 11 and including processing means for processing signals from said at least one ultrasonic receiver unit and for identifying a receipt time of said longitudinal wave.

13. Apparatus according to claim 12 and wherein said processing means includes means for identifying receipt times of said surface and transverse waves.

14. Apparatus according to claim 12 and wherein said processing means includes means for determining said mechanical properties from at least said receipt time for said longitudinal wave.

15. Apparatus according to claim 13 and wherein said processing means includes means for determining said mechanical properties from said receipt times of said surface and transverse waves.

16. Apparatus according to claim 1 and wherein said transmission means transmits said ultrasonic wave within a generally small range of a predetermined angle.

17. Apparatus according to claim 1 and wherein a transmission surface of said transmission means and effective receiving locations of said receiving means are generally coplanar.

18. Apparatus according to claim 1 and characterized in that generally no knowledge of properties of said interposed medium are necessary.

19. A method for determining, through an interposed medium, the mechanical properties of a solid having a surface, the method comprising the steps of:

transmitting ultrasonic waves through said interposed medium and through said solid and generally parallel to said surface from a first location;
receiving said ultrasonic waves with at least one ultrasonic receiver unit located in each of second and third locations, wherein said first, second and third locations are colinear along said surface;
locating said at least one receiver unit such that a first receipt time of an ultrasonic wave from said surface to said at least one ultrasonic receiver unit at said second location is generally equivalent to a second receipt time from said surface to said at least one receiver unit at said third location, and
calculating said mechanical properties from ultrasonic waves transmitted by said ultrasonic transmission means once said first and second receipt times are generally equivalent.

20. Method according to claim 19 and wherein said step of locating includes the step of adjusting the locations of said at least one receiver unit such that said first and second receipt times are generally equivalent.

21. Method according to claim 20 and wherein said step of locating includes the step of determining said first and said second receipt times.

22. Method according to claim 19 and wherein said step of determining includes the step of pulsing said transmission means thereby to transmit ultrasonic waves when said first and second receipt times are generally equivalent.

23. Method according to claim 19 and wherein said solid is in vivo bone.

24. Method according to claim 19 and wherein said solid is selected from the group of metal, plastics and wood.

25. Method according to claim 19 and wherein the velocity of said ultrasonic waves through said interposed medium is less than the velocity of said ultrasonic waves through said solid.

26. Method according to claim 19 and wherein said at least one ultrasonic receiver unit is placed onto a surface of said interposed medium generally without any coupling medium.

27. A method according to any of claims 19-26, and wherein: said at least one ultrasonic receiver unit comprises at least first and second ultrasonic receiver units; and said step of locating said at least one ultrasonic receiver unit includes

using a rocker unit to rockingly press said ultrasonic receiver units into said interposed medium thereby adjusting the locations of said receiver units such that said first and second receipt times are generally equivalent.

28. Method according to claim 19 and including the step of transmitting said ultrasonic waves in a wide band pattern and wherein said ultrasonic waves generate longitudinal, surface and transverse waves in said solid.

29. Method according to claim 28 and including the steps of processing signals from said at least one ultrasonic receiver unit and identifying a receipt time of said longitudinal wave.

30. Method according to claim 29 and wherein said step of processing includes the step of identifying receipt times of said surface and transverse waves.

31. Method according to claim 29 and wherein said step of processing includes the step of determining said mechanical properties from at least said receipt time for said longitudinal wave.

32. Method according to claim 30 and wherein said step of processing includes the step of determining said mechanical properties from said receipt times of said surface and transverse waves.

33. Apparatus according to claim 2 and wherein said means for locating include a rocker unit for rockingly pressing said receiver units into said interposed medium.

34. Apparatus according to claim 33 and wherein said rocker unit comprises an acoustically insulating unit.

35. Method according to claim 20 and wherein said step of locating includes the step of using a rocker unit to rockingly press said at least one ultrasonic receiver unit into said interposed medium.

36. Apparatus according to claim 1 wherein said solid comprises more than one material and wherein said means for calculating include:

means for receiving a time-based output signal from each of said at least one ultrasonic receiver unit;
means for transforming at least two of said output signals to the frequency domain thereby to produce frequency domain signals; and
means for determining, from said frequency domain signals, frequency values and their corresponding propagation speeds, for each of said materials within said solid.

37. Apparatus according to claim 36 and also comprising means for determining, from a difference of said frequency domain signals, extent of attenuation of each of said materials within said solid.

38. Apparatus according to claim 1 and including means for receiving a time-based output signal from each of said at least one ultrasonic receiver unit and determining a first peak of each of said output signals and means for calculating from said first peaks, the extent of attenuation of said ultrasonic waves by said solid.

39. Apparatus according to claim 1 and wherein said means for calculating includes means for estimating a thickness of said solid.

40. Apparatus according to either of claims 1 or 9 and also including a housing and an acoustic barrier wherein said ultrasonic transmission means are located on a first side of said acoustic barrier and said at least one ultrasonic receiver unit is located on a second side of said acoustic barrier.

41. Apparatus according to claim 1 and wherein said means for locating include:

a housing in which said at least one ultrasonic receiver unit is located;
a flexible membrane attached to said housing at one end and forming a closed section and an open section, said closed section being filled with a coupling material, said flexible membrane having a vacuum hole at a second end thereof,
wherein, when said apparatus is placed onto human skin and air within said vacuum hole is evacuated, said apparatus is sealingly coupled with said human skin.

42. A method according to claim 19 wherein said solid comprises more than one material and wherein said step of calculating includes the steps of:

receiving a time-based output signal from each of said at least one ultrasonic receiver unit;
transforming at least two of said output signals to the frequency domain thereby to produce frequency domain signals; and
determining, from said frequency domain signals, frequency values and their corresponding propagation speeds, for each of said materials within said solid.

43. A method according to claim 42 and also including the step of determining, from a difference of said frequency domain signals, extent of attenuation of each of said materials within said solid.

44. A method according to claim 19 and including the steps of receiving a time-based output signal from each of said at least one ultrasonic receiver unit and determining a first peak of each of said output signals and calculating from said first peaks, the extent of attenuation of said ultrasonic waves by said solid.

45. A method according to claim 19 and wherein said step of calculating includes the step of estimating a thickness of said solid.

**Patentansprüche**

1. Vorrichtung zum Bestimmen der mechanischen Eigenschaften eines festen Körpers (16), der eine Oberfläche (18) besitzt, durch ein zwischengesetztes Medium (20), wobei die Vorrichtung umfasst:

    in einem ersten Ort angeordnete Ultraschall-Sendemittel (10, 60) zum Senden von Ultraschallwellen durch das zwischengesetzte Medium und den festen Körper und allgemein parallel zu der Oberfläche;

    mindestens eine Ultraschall-Empfängereinheit (12a, 12b, 64), die in jedem von zweiten und dritten Orten zum Empfangen der Ultraschallwellen angeordnet ist, wobei der erste, der zweite und der dritte Ort längs der Oberfläche kolinear sind;

    Mittel zum Anordnen (61) der mindestens einen Empfängereinheit in solcher Weise, dass eine erste Empfangs zeit einer Ultraschallwelle von der Oberfläche zu dem der mindestens einen Ultraschall-Empfängereinheit an der zweiten Stelle allgemein äquivalent einer zweiten Empfangs zeit von der Oberfläche zu der mindestens einen Ultraschall-Empfängereinheit an dem dritten Ort äquivalent ist; und

    Mittel zum Berechnen (61) der mechanischen Eigenschaften von durch das Ultraschall-Sendemittel gesendeten Ultraschallwellen, sobald die erste und die zweite Empfangs zeit allgemein äquivalent sind.

2. Vorrichtung nach Anspruch 1 und bei der das Mittel zum Lokalisieren die Orte der mindestens einen Empfängereinheit so einstellt, dass die erste und zweite Empfangs zeit allgemein äquivalent sind.

3. Vorrichtung nach Anspruch 2 und bei der das Mittel zum Lokalisieren Mittel zum Bestimmen der ersten und der zweiten Empfangszeit enthält.

4. Vorrichtung nach Anspruch 3 und bei der das Mittel zum Bestimmen Mittel zum Pulsieren des Sendemittels enthält, um dadurch Ultraschallwellen zu übertragen, wenn die erste und die zweite Empfangszeit allgemein identisch sind.

5. Vorrichtung nach Anspruch 1 und bei der der feste Körper ein Knochen im lebenden Körper ist.

6. Vorrichtung nach Anspruch 1 und bei der der feste Körper aus der Gruppe von Metall, Kunststoff und Holz ausgewählt ist.

7. Vorrichtung nach Anspruch 1 und bei der die Geschwindigkeit der Ultraschallwellen durch das zwischengesetzte Medium geringer als die Geschwindigkeit der Ultraschallwellen durch den festen Körper ist.

8. Vorrichtung nach Anspruch 1 und bei der die mindestens eine Ultraschall-Empfängereinheit auf eine Oberfläche des zwischengesetzten Mediums allgemein ohne jedes Kopplungsmedium aufgesetzt ist.

9. Vorrichtung nach einem der vorangehenden Ansprüche und bei der:

    die mindestens eine Ultraschall-Empfängereinheit mindestens erste und zweite Ultraschall-Empfängereinheiten umfasst; und

    das Mittel zum Lokalisieren der Ultraschall-Empfängereinheiten eine Walkeinheit zum walkenden Eindrücken der Ultraschall-Empfängereinheiten in das zwischengesetzte Medium enthält, so dass eine erste Empfangszeit der Ultraschallwelle von der Oberfläche zu der ersten Ultraschall-Empfängereinheit allgemein äquivalent einer zweiten Empfangszeit von der Oberfläche zu der zweiten Ultraschall-Empfängereinheit ist.

10. Vorrichtung nach Anspruch 9 und bei der die Walkeinheit eine akustisch isolierende Einheit umfasst.

11. Vorrichtung nach Anspruch 1, bei der das Ultraschall-Sende mittel Mittel zum Senden der Ultraschallwelle in einem breiten Winkel enthält, und bei der die Ultraschallwellen Längs-, Oberflächen- und Querwellen in dem festen Körper erzeugen.

12. Vorrichtung nach Anspruch 11, und die Verarbeitungsmittel zum Verarbeiten von Signalen von der mindestens einen Ultraschall-Empfängereinheit und zum Identifizieren einer Empfangszeit der Längswelle enthält.

13. Vorrichtung nach Anspruch 12, und bei der das Verarbeitungsmittel Mittel zum Identifizieren der Empfangs zeitpunkte der Oberflächen- und Querwellen enthält.

14. Vorrichtung nach Anspruch 12, und bei der das Verarbeitungsmittel Mittel zum Bestimmen der mechanischen Eigenschaften aus mindestens der Empfangs zeit für die Längswelle enthält.

15. Vorrichtung nach Anspruch 13 und bei der das Verarbeitungsmittel Mittel zum Bestimmen der mechanischen Eigenschaften aus den Empfangszeiten der Oberflächen- und Querwellen enthält.

16. Vorrichtung nach Anspruch 1 und bei der das Sendemittel die Ultraschallwelle innerhalb eines allgemein kleinen Bereiches mit einem vorgegebenen Winkel überträgt.

17. Vorrichtung nach Anspruch 1 und bei der eine Sendefläche des Sendemittels und effektive Empfangsorte des Empfängermittels allgemein koplanar sind.

18. Vorrichtung nach Anspruch 1 und **dadurch gekennzeichnet,** dass allgemein keine Kenntnis von Eigenschaften des zwischengesetzten Mediums notwendig ist.

19. Verfahren zum Bestimmen der mechanischen Eigenschaften eines festen Körpers, der eine Oberfläche besitzt, durch ein zwischengesetztes Medium, wobei das Verfahren die Schritte umfasst:

Senden von Ultraschallwellen durch das zwischengesetzte Medium und durch den festen Körper und allgemein parallel zu der Oberfläche von einem ersten Ort aus;

Empfangen der Ultraschallwellen mit mindestens einer Ultraschall-Empfängereinheit, die an jedem von zweiten und dritten Orten gelegen ist, wobei die ersten, zweiten und dritten Orte längs der Oberfläche kolinear sind;

Lokalisieren der mindestens einen Empfängereinheit in der Weise, dass eine erste Empfangszeit einer Ultraschallwelle von der Oberfläche zu der mindestens einen Ultraschall-Empfängereinheit an dem zweiten Ort allgemein äquivalent zu einer zweiten Empfangs zeit von der Oberfläche zu der mindestens einen Empfängereinheit an dem dritten Ort ist, und

Berechnen der mechanischen Eigenschaften aus Ultraschallwellen, die durch das Ultraschall-Sendemittel übertragen wurden, sobald die erste und die zweite Empfangs zeit allgemein äquivalent sind.

20. Verfahren nach Anspruch 19 und bei dem der Schritt des Lokalisierens den Schritt des Einrichtens der Orte der mindestens einen Empfängereinheit enthält, so dass die ersten und die zweiten Empfangszeiten allgemein äquivalent sind.

21. Verfahren nach Anspruch 20 und bei dem der Schritt des Lokalisierens den Schritt des Bestimmens der ersten und der zweiten Empfangszeiten enthält.

22. Verfahren nach Anspruch 19 und bei dem der Schritt des Bestimmens den Schritt des Pulsierens des Sendemittels enthält, um dadurch Ultraschallwellen zu übertragen, wenn die erste und die zweite Empfangs zeit allgemein äquivalent sind.

23. Verfahren nach Anspruch 19, und bei dem der feste Körper ein Knochen im lebenden Körper ist.

24. Verfahren nach Anspruch 19 und bei dem der feste Körper aus der Gruppe von Metall, Kunststoff und Holz ausgewählt ist.

25. Verfahren nach Anspruch 19 und bei dem die Geschwindigkeit der Ultraschallwellen durch das zwischengesetzte Medium geringer als die Geschwindigkeit der Ultraschallwellen durch den festen Körper ist.

26. Verfahren nach Anspruch 19 und bei dem die mindestens eine Ultraschall-Empfängereinheit auf eine Oberfläche des zwischengesetzten Mediums allgemein ohne jedes Kupplungsmedium aufgesetzt ist.

27. Verfahren nach einem der Ansprüche 19 bis 26 und bei dem:

die mindestens eine Ultraschall-Empfängereinheit mindestens erste und zweite Ultraschall-Empfängereinheiten umfasst; und

der Schritt des Lokalisierens der mindestens einen Ultraschall-Empfängereinheit umfasst:

das Benutzen einer Walkeinheit zum walkenden Eindrücken der Ultraschall-Empfängereinheiten in das zwischengesetzte Medium, um dadurch die Orte der Empfängereinheiten so einzustellen, dass die erste und die zweite Empfangs zeit allgemein äquivalent sind.

28. Verfahren nach Anspruch 19, und das den Schritt des Sendens der Ultraschallwellen in einer Breitbandverteilung umfasst, und wobei die Ultraschallwellen Längs-, Oberflächen- und Querwellen in dem festen Körper erzeugen.

29. Verfahren nach Anspruch 28, und das die Schritte des Verarbeitens von Signalen von der mindestens einen Ultraschall-Empfängereinheit und des Identifizierens einer Empfangszeit der Längswellen enthält.

30. Verfahren nach Anspruch 29, und bei dem der Schritt des Verarbeitens den Schritt des Identifizierens von Empfangszeiten der Oberflächen- und Querwellen enthält.

31. Verfahren nach Anspruch 29, und bei dem der Schritt des Verarbeitens den Schritt des Bestimmens der mechanischen Eigenschaften mindestens aus der Empfangs zeit für die Längswelle enthält.

32. Verfahren nach Anspruch 30, und bei dem der Schritt des Verarbeitens den Schritt des Bestimmens der mechanischen Eigenschaften aus den Empfangs zeiten der Oberflächen- und Querwellen enthält.

33. Vorrichtung nach Anspruch 2, und bei der das Mittel zum Lokalisieren eine Walkeinheit zum walkenden Eindrücken der Empfängereinheiten in das zwischengesetzte Medium enthält.

34. Vorrichtung nach Anspruch 33 und bei der die Walkeinheit eine akustische Isoliereinheit umfasst.

35. Verfahren nach Anspruch 20 und bei dem der Schritt des Lokalisierens den Schritt des Benutzens einer Walkeinheit zum walkenden Eindrücken der mindestens einen Ultraschall-Empfängereinheit in das zwischengesetzte Medium enthält.

36. Vorrichtung nach Anspruch 1, und bei der feste Körper mehr als ein Material umfasst und die Mittel zum Berechnen enthalten:

Mittel zum Empfangen eines auf Zeit basierenden Ausgangssignals von jeder der mindestens einen Ultraschall-Empfängereinheit;

Mittel zum Umformen von mindestens zweien der Ausgangssignale in die Frequenzabhängigkeit, um dadurch frequenzabhängig aufgezeichnete Signale zu erzeugen; und

Mittel zum Bestimmen aus den frequenzabhängigen Signalen von Frequenzwerten und ihre entsprechenden Fortpflanzung-Geschwindigkeiten für jedes der Materialien innerhalb des festen Körpers.

37. Vorrichtung nach Anspruch 36, und die auch Mittel zum Bestimmen des Ausmaßes der Dämpfung jedes der Materialien innerhalb des Massivteils aus einer Differenz der frequenzabhängig aufgezeichneten Signale umfasst.

**38.** Vorrichtung nach Anspruch 1, und die Mittel zum Empfangen eines auf Zeit basierenden Ausgangssignales von jedem der mindestens einen Ultraschall-Empfängereinheit und zum Bestimmen einer ersten Spitze jedes der Ausgangssignale und Mittel zum Berechnen des Ausmaßes der Dämpfung der Ultraschallwellen durch den festen Körper aus den ersten Spitzen enthält.

**39.** Vorrichtung nach Anspruch 1, und bei der das Mittel zum Berechnen Mittel zum Abschätzen einer Dicke des festen Körpers enthält.

**40.** Vorrichtung nach einem der Ansprüche 1 bis 9, und die auch ein Gehäuse und eine Akustiksperre enthält, wobei das Ultraschall-Sendemittel an einer ersten Seite der akustischen Sperre und die mindestens eine Ultraschall-Empfängereinheit an einer zweiten Seite der akustischen Sperre gelegen ist.

**41.** Vorrichtung nach Anspruch 1, und bei der die Mittel zum Lokalisieren enthalten:

ein Gehäuse, in welchem die mindestens eine Ultraschall-Empfängereinheit gelegen ist;

eine flexible Membran, die an dem Gehäuse an einem Ende angebracht ist und einen geschlossenen und einen offenen Abschnitt bildet, wobei der geschlossene Abschnitt mit einem Kupplungsmaterial gefüllt ist, die flexible Membran eine Vakuumöffnung an einem zweiten Ende derselben besitzt,

wobei, wenn die Vorrichtung auf menschliche Haut aufgesetzt und Luft in der Vakuumöffnung evakuiert wird, die Vorrichtung dichtend mit der menschlichen Haut gekoppelt wird.

**42.** Verfahren nach Anspruch 19, bei dem der feste Körper mehr als ein Material umfasst und bei dem der Berechnungsschritt die Schritte enthält:

des Empfangens eines Zeitbasis-Ausgangssignals von jeder der mindestens einen Ultraschall-Empfängereinheit;

des Umformens von mindestens zweien der Ausgangssignale in die frequenzabhängige Aufzeichnung, um dadurch frequenzabhängig aufgezeichnete Signale zu erzeugen; und

des Bestimmens von Frequenzwerten und ihren entsprechenden Fortpflanzungs- Geschwindigkeiten für jedes der Materialien innerhalb des Massivteils aus den frequenzabhängig aufgezeichneten Signalen.

**43.** Verfahren nach Anspruch 42, und das auch den Schritt des Bestimmens des Ausmaßes der Dämpfung in jedem der Materialien innerhalb des festen Körpers aus einer Differenz der frequenzabhängig aufgezeichnetenen Signale enthält.

**44.** Verfahren nach Anspruch 19, und das die Schritte enthält des Empfangens eines Zeitbasis-Ausgangssignals von jeder der mindestens einen Ultraschall-Empfängereinheit und des Bestimmens eines ersten Gipfels jedes der Ausgangssignale und des Errechnens des Ausmaßes der Dämpfung der Ultraschallwellen durch den festen Körper aus den ersten Gipfeln.

**45.** Verfahren nach Anspruch 19, und bei dem der Schritt des Berechnens den Schritt des Abschätzens einer Dicke des festen Körpers enthält.

**Revendications**

**1.** Appareil pour déterminer, par l'intermédiaire d'un milieu intercalé (20), des propriétés mécaniques d'un solide (16) possédant une surface (18), l'appareil comprenant :

des moyens de transmission d'ultrasons (10, 60) situés en un premier emplacement pour transmettre des ondes ultrasoniques à travers ledit milieu intercalé et à travers ledit solide et d'une manière générale parallèlement à ladite surface ;
au moins une unité formant récepteur d'ultrasons (12a, 12b, 64) située en chacun de second et troisième emplacements pour recevoir lesdites ondes ultrasoniques, lesdits premier, second et troisième emplacements étant colinéaires le long de ladite surface ;

des moyens pour localiser (61) ladite au moins une unité formant récepteur de sorte qu'un première durée de réception d'une onde ultrasonique provenant de ladite surface sur ladite au moins une unité formant récepteur d'ultrasons audit second emplacement est sensiblement équivalent d'une manière générale à une seconde durée de réception depuis ladite surface jusqu'à ladite au moins une unité formant récepteur d'ultrasons audit troisième emplacement ; et

des moyens pour calculer (61) lesdites propriétés mécaniques à partir d'ondes ultrasoniques transmises par lesdits moyens de transmission d'ultrasons une fois que lesdites première et seconde durées de réception sont sensiblement équivalentes.

2. Appareil selon la revendication 1 et dans lequel lesdits moyens de localisation règlent les emplacements de ladite au moins une unité formant récepteur de telle sorte que lesdites première et seconde durées de réception sont sensiblement équivalentes.

3. Appareil selon la revendication 2 et dans lequel lesdits moyens de localisation incluent des moyens pour déterminer lesdites première et seconde durées de réception.

4. Appareil selon la revendication 3 et dans lequel lesdits moyens de détermination incluent des moyens pour commander d'une manière impulsionnelle lesdits moyens de transmission de manière à transmettre les ondes ultrasoniques lorsque lesdites première et seconde durées sont sensiblement identiques.

5. Appareil selon la revendication 1 et dans lequel ledit solide se situe dans un os vivant.

6. Appareil selon la revendication 1 et dans lequel ledit solide est choisi dans le groupe incluant un métal, une matière plastique et le bois.

7. Appareil selon la revendication 1 et dans lequel la vitesse desdites ondes ultrasoniques à travers ledit milieu intercalé est inférieure à la vitesse desdites ondes ultrasoniques à travers ledit solide.

8. Appareil selon la revendication 1 et dans lequel ladite au moins une unité formant récepteur d'ultrasons est disposée sur une surface dudit milieu intercalé, d'une manière générale sans aucun milieu de couplage.

9. Appareil selon l'une quelconque des revendications précédentes et dans lequel ladite au moins une unité formant récepteur d'ultrasons comprend :

au moins des première et seconde unités formant récepteurs d'ultrasons ; et

lesdits moyens pour localiser lesdites unités formant récepteurs d'ultrasons comprennent une unité basculante servant à serrer avec possibilité de basculement lesdites unités formant récepteurs d'ultrasons dans ledit milieu intercalé de sorte qu'une première durée de réception de ladite onde ultrasonique depuis ladite surface jusqu'à ladite première unité formant récepteur d'ultrasons est sensiblement équivalente, d'une manière générale à une seconde durée de réception depuis ladite surface jusqu'à ladite seconde unité formant récepteur d'ultrasons.

10. Appareil selon la revendication 9 et dans lequel ladite unité basculante comprend une unité d'isolation acoustique.

11. Appareil selon la revendication 1, dans lequel lesdites moyens de transmission ultrasonique comprennent des moyens pour transmettre ladite onde ultrasonique dans un angle large et dans lequel lesdites ondes ultrasoniques produisent des ondes longitudinales, des ondes de surface et des ondes transversales dans ledit solide.

12. Appareil selon la revendication 11 et comprenant des moyens de traitement pour traiter des signaux provenant de ladite au moins une unité formant récepteur d'ultrasons et pour identifier une durée de réception de ladite onde longitudinale.

13. Appareil selon la revendication 12 et dans lequel lesdits moyens de traitement incluent des moyens pour identifier des durées de réception desdites ondes de surface et desdites ondes transversales.

14. Appareil selon la revendication 12 et dans lequel lesdits moyens de protection incluent des moyens pour déterminer lesdites propriétés chimiques à partir au moins de ladite durée de réception pour ladite onde longitudinale.

**15.** Appareil selon la revendication 13 et dans lequel lesdits moyens de traitement comprennent des moyens pour déterminer lesdites propriétés mécaniques à partir desdites durées de réception desdites ondes de surface et desdites ondes transversales.

**16.** Appareil selon la revendication 1 et dans lequel lesdits moyens de transmission transmettent ladite onde ultrasonique dans une gamme en général faible d'un angle prédéterminé.

**17.** Appareil selon la revendication 1 et dans lequel une surface de transmission desdits moyens de transmission et des emplacements de réception effective desdits moyens de réception sont en général coplanaires.

**18.** Appareil selon la revendication 1 et caractérisé en ce que d'une manière générale aucune connaissance de propriétés dudit milieu intercalé n'est nécessaire.

**19.** Procédé pour déterminer, au moyen d'un milieu intercalé, les propriétés mécaniques d'un solide possédant une surface, le procédé comprenant les étapes consistant à :

transmettre les ondes ultrasoniques à travers ledit milieu intercalé et à travers ledit solide et d'une manière générale parallèlement à ladite surface à partir d'un premier emplacement ;
recevoir lesdites ondes ultrasoniques à l'aide d'au moins une unité formant récepteur d'ultrasons située dans chacun desdits second et troisième emplacements, lesdits premier, second et troisième emplacements étant colinéaires le long de ladite surface ;
localiser ladite au moins une unité formant récepteur de sorte qu'une première durée de réception d'une onde ultrasonique provenant de ladite surface sur ladite au moins une unité formant récepteur d'ultrasons audit second emplacement est sensiblement équivalent d'une manière générale à une seconde durée de réception depuis ladite surface jusqu'à ladite au moins une unité formant récepteur d'ultrasons audit troisième emplacement ; et
calculer lesdites propriétés mécaniques à partir d'ondes ultrasoniques transmises par lesdits moyens de transmission d'ultrasons une fois que lesdites première et seconde durées de réception sont sensiblement équivalentes.

**20.** Procédé selon la revendication 19 et dans lequel ladite étape de localisation inclut l'étape de réglage des positions de ladite au moins une unité formant récepteur de telle sorte que lesdites première et seconde durées de réception sont sensiblement équivalentes.

**21.** Procédé selon la revendication 20, et selon lequel ladite étape de positionnement inclut l'étape de détermination desdites première et seconde durées de réception.

**22.** Procédé selon la revendication 19, et selon lequel ladite étape de détermination inclut l'étape de commande impulsionnelle desdits moyens de transmission afin de transmettre des ondes ultrasoniques lorsque lesdites première et seconde durées de réception sont sensiblement équivalentes.

**23.** Procédé selon la revendication 19 et selon lequel ledit solide est un os vivant.

**24.** Procédé selon la revendication 19 et dans lequel ledit solide est choisi dans le groupe comprenant un métal, une matière plastique et du bois.

**25.** Procédé selon la revendication 19 et selon lequel la vitesse desdites ondes ultrasoniques dans ledit milieu intercalé est inférieure à la vitesse des ondes ultrasoniques dans ledit solide.

**26.** Procédé selon la revendication 19 et selon lequel ladite au moins une unité formant récepteur d'ultrasons est placée sur une surface dudit milieu intercalé, d'une manière générale sans aucun milieu de couplage.

**27.** Procédé selon l'une quelconque des revendications 19-26 et selon lequel :

ladite au moins une unité formant récepteur d'ultrasons comprend au moins les première et seconde unités formant récepteurs d'ultrasons ; et
que ladite étape de localisation de ladite au moins une unité formant récepteur d'ultrasons comprend l'utilisation d'une unité basculante servant à serrer, avec possibilité de basculement lesdites unités formant récepteurs d'ultrasons dans ledit milieu intercalé de manière à régler les positions desdites unités formant récepteurs d'ultrasons de telle sorte que lesdites première et seconde durées de réception sont sensiblement équivalentes.

28. Procédé selon la revendication 19 et incluant l'étape consistant à transmettre lesdites ondes ultrasoniques dans une configuration à large bande, et selon lequel lesdites ondes ultrasoniques produisent des ondes longitudinales, des ondes de surface et des ondes transversales dans ledit solide.

29. Procédé selon la revendication 28 et incluant les étapes de traitement de signaux provenant de ladite au moins une unité formant récepteur d'ultrasons et d'identification d'une durée de réception de ladite onde longitudinale.

30. Procédé selon la revendication 29 et selon lequel ladite étape de traitement inclut l'étape d'identification de durées de réception desdites ondes de surface et desdites ondes transversales.

31. Procédé selon la revendication 29 et selon lequel ladite étape de traitement inclut l'étape de détermination desdites propriétés mécaniques à partir au moins de la durée de réception pour ladite onde longitudinale.

32. Procédé selon la revendication 30 et selon lequel ladite étape de traitement inclut l'étape de détermination desdites propriétés mécaniques à partir desdites durées de réception desdites ondes de surface et desdites ondes transversales.

33. Appareil selon la revendication 2 et selon lequel lesdits moyens de localisation incluent une unité basculante servant à serrer, avec possibilité de basculement, lesdites unités formant récepteurs dans ledit milieu intercalé.

34. Appareil selon la revendication 23 et dans lequel ladite unité basculante comprend une unité d'isolation acoustique.

35. Appareil selon la revendication 20 et selon laquelle ladite étape de localisation inclut l'étape consistant à utiliser une unité basculante pour serrer, avec possibilité de basculement, au moins une unité formant récepteur d'ultrasons dans ledit milieu intercalé.

36. Appareil selon la revendication 1, selon lequel ledit solide comprend plus d'un matériau et selon lequel lesdits moyens de calcul comprennent :

des moyens pour recevoir un signal de sortie basé sur un temps délivré par chacune desdites unités formant récepteurs d'ultrasons ;
des moyens pour transformer deux desdits signaux de sortie en les amenant dans le domaine des fréquences de manière à produire des signaux du domaine des fréquences ; et
des moyens pour déterminer, à partir desdits signaux du domaine des fréquences, des valeurs de fréquence et leurs vitesses de propagation correspondantes, pour chacun desdits matériaux à l'intérieur dudit solide.

37. Appareil selon la revendication 36 et comprenant également des moyens pour déterminer, à partir d'une différence desdits signaux du domaine des fréquences, un degré d'atténuation de chacun desdits matériaux dans ledit solide.

38. Appareil selon la revendication 1 et comprenant des moyens pour recevoir un signal de sortie basé sur un temps provenant de la ou chacune desdites unités formant récepteurs d'ultrasons et pour déterminer un premier pic de chacun desdits signaux de sortie, et des moyens pour calculer, à partir desdits premiers pics, le degré d'atténuation desdites ondes ultrasoniques par ledit solide.

39. Appareil selon la revendication 1 et dans lequel lesdits moyens de calcul comprennent des moyens pour estimer une épaisseur dudit solide.

40. Appareil selon l'une ou l'autre des revendications 1 ou 9, et également comprenant un boîtier et une barrière acoustique, dans lequel lesdits moyens de transmission ultrasoniques sont situés sur un premier côté de ladite barrière acoustique et ladite au moins une unité formant récepteur d'ultrasons est située sur un second côté de ladite barrière acoustique.

41. Appareil selon la revendication 1 et dans lequel lesdits moyens de localisation incluent :

un boîtier, dans lequel ladite au moins une unité formant récepteur d'ultrasons est située ;

une membrane flexible fixée à une extrémité audit boîtier et formant une section fermée et une section ouverte, ladite section fermée étant remplie par un matériau de couplage, ladite membrane flexible possédant, à une seconde extrémité, un trou d'application de vide,

auquel cas, lorsque ledit appareil est placé sur une peau humaine et que de l'air à l'intérieur dudit tube à vide est évacué, ledit appareil est accouplé d'une manière étanche à ladite peau humaine.

42. Procédé selon la revendication 19, selon lequel ledit solide comprend plus d'un matériau, et selon lequel ladite étape de calcul comprend les étapes consistant à :

recevoir un signal de sortie basé sur un temps à partir de la ou de chacune desdites unités formant récepteurs d'ultrasons ;

transformer au moins deux desdits signaux de sortie pour l'amener dans le domaine des fréquences, de manière à produire des signaux dans le domaine des fréquences ; et

déterminer, à partir desdits signaux du domaine des fréquences, des valeurs de fréquence et leurs vitesses correspondantes de propagation, pour chacun desdits matériaux à l'intérieur dudit solide.

43. Procédé selon la revendication 42 et comprenant également l'étape de détermination, à partir d'une différence desdits signaux du domaine des fréquences, le degré d'atténuation de chacun desdits matériaux dans ledit solide.

44. Procédé selon la revendication 19 et incluant les étapes pour recevoir un signal de sortie basé sur un temps provenant de la ou desdites unités formant récepteurs d'ultrasons et pour déterminer un premier pic de chacun desdits signaux de sortie, et des moyens pour calculer, à partir desdits premiers pics, le degré d'atténuation desdites ondes ultrasoniques par ledit solide.

45. Procédé selon la revendication 19 et selon lequel ladite étape de calcul inclut l'étape d'estimation d'une épaisseur dudit solide.

FIG.1

EP 0 585 492 B1

FIG.2A

FIG.2B

FIG.3

EP 0 585 492 B1

FIG.4

FIG.5

FIG.6

FIG.7

FIG.8

EP 0 585 492 B1

FIG.9

```
            STORE  F1

            STORE  F2

         FFT(F1)  =  SF1
         FFT(F2)  =  SF2

        FIND  PEAKS  SF1
        FIND  PEAKS  SF2

        RESCALE  SF1
        RESCALE  SF2        ◄────  V_L
        WITH  VELOCITY

           LOG(SF1)
           LOG(SF2)

  20LOG(SF1)  −  20LOG(SF2)  =  ATTENUATION
```

FIG.10

FIG.11A

FIG.11B

FIG.12

FIG.13

FIG. 14

FIG.15

FIG.16

FIG.17

FIG.18B

FIG.18A